# EUROPEAN PATENT SPECIFICATION

(11) **EP 2 550 360 B1**
(45) Date of publication and mention of the grant of the patent: **30.08.2017**
(21) Application number: 11711512.1
(22) Date of filing: 24.03.2011
(51) Int. Cl.: C12N 15/113, A61K 31/712

(54) **BIVALENT ANTISENSE OLIGONUCLEOTIDES**
BIVALENTE ANTISENSE-OLIGONUKLEOTIDE
OLIGONUCLÉOTIDES ANTISENS BIVALENTS

(30) Priority: 24.03.2010 DK 201000241
(43) Date of publication of application: 30.01.2013
(73) Proprietor: Mirrx Therapeutics A/s, 2800 Lyngby (DK)
(72) Inventor: MOELLER, Thorleif, S-216 14 Limhamn (SE); UDESEN, Christina, S-216 14 Limhamn (SE)
(74) Representative: Orsnes, Henrik Egede
(86) International application number: PCT/EP2011/054545
(87) International publication number: WO 2011/117353

(56) References cited:
- WO-A1-2011/031520
- WO-A2-2005/013901
- WO-A2-2005/078096
- WO-A2-2005/111238
- WO-A2-2007/112754
- WO-A2-2008/061537
- WO-A2-2008/151639
- WO-A2-2009/023819

## Description

### Background

MicroRNAs are small noncoding RNA that bind to microRNA binding sites in target RNA to impose translational regulation or altered stability of the target RNA. Typically, the activity of the target RNA is decreased either because the microRNA destabilizes the target RNA to which it binds or because microRNA binding to the target RNA leads to translational repression.

Currently, it is estimated that between 500 and 1000 human microRNA exist and it is estimated that more than 50% of all human genes are subject to microRNA regulation. A specific microRNA may bind to and regulate a large number of target RNAs (typically mRNAs) e.g. up to 100 target RNA. Moreover, a specific target RNA may comprise several microRNA binding sites for identical or different microRNAs. When several microRNAs bind to the same target RNA, they often bind cooperatively.

Given the number of microRNA and also the number of genes estimated to be regulated by microRNAs, it is expected that microRNAs play a role in many, if not most gene regulatory processes and also in disease development and disease states. Indeed, it is becoming increasingly clear that microRNAs play a role in many diseases.

Therefore, there is great interest in being able to modulate microRNA regulatory pathways.

Fundamentally, two ways of negatively affecting microRNA regulatory pathways may be contemplated.

First, microRNAs may be inactivated, e.g. by molecules that bind directly to microRNAs. This approach has been used almost since microRNAs were discovered. Thus, already in 2003 steric blockers binding to microRNA was described (also termed antimirs or antagomirs). The consequence of such an approach is that all target RNAs of a given microRNA is deregulated.

A second approach was described in WO2008/061537. This approach employs so-called Blockmirs that bind to microRNA binding sites in target RNAs. Thus, Blockmirs enable specific deregulation of one specific microRNA target of a given microRNA, while allowing the microRNA to regulate all its other targets.

WO 2005/078096 discloses an RNA-silencing agent comprising a mRNA targeting moiety, a linking moiety, and miRNA recruiting moiety. Thus, the RNA-silencing agent can bind to a mRNA with one moiety and bring the RISC complex to the mRNA via the microRNA recruiting moiety leading to repression of the mRNA. WO 2005/078096 does not describe agents that prevent translational repression of mRNA.

While Blockmirs and antimirs are very important molecules that can be used to modulate microRNA regulatory pathways, they have some shortcomings. Thus, an antimir as described in the state of the art cannot simultaneously bind to two different or identical microRNAs (or even microRNA families) which may be desirable in some situations.

Moreover, a Blockmirs as described in the prior art cannot simultaneously bind to two microRNA bindings sites, which may be desirable in some situations.

### Brief description of the drawings

Figure 1.
   Dual luciferase measurements of the activity of bivalent molecules of the invention, refer to example 4 for details.
Figure 2.
   Dual luciferase measurements of the activity of bivalent molecules of the invention, refer to example 4 for details.
Figure 3.
   Dual luciferase measurements of the activity of bivalent molecules of the invention, refer to example 5 for details.
Figure 4.
   Dual luciferase measurements of the activity of bivalent molecules of the invention, refer to example 5 for details.

### Disclosure of the invention

### Definitions and terms

When referring to the "activity of a target mRNA", what is typically meant is the expression of the target mRNA, i.e. translation into a protein or peptide. Thus, regulation of the activity of a target mRNA may include degradation of the mRNA and/or translational regulation. The activity may also be replication.

The terms "regulated" and "modulate" are used interchangeably herein.

As used herein, regulation may be either positive or negative. I.e. a regulator (e.g. oligonucleotide or microRNA) may increase the activity of the target (e.g. target mRNA) or it may decrease the activity of the target.

When the target RNA is a viral RNA, the molecules of the invention may affect replication of the virus or otherwise interfere with the proliferation of the virus.

The term microRNA as used herein has the same meaning as typically in the art. I.e. the term microRNA refers to a small non-translated RNA of typically 18-22 nucleotides that is capable of regulating the activity of a target mRNA. A microRNA is typically processed from pri-microRNA to short stem-loop structures called pre-microRNA and finally to mature miRNA. Both strands of the stem of the pre-microRNA may be processed to a mature microRNA.

The miRBase (http://microrna.sanger.ac.uk/sequences/) is a compilation of known microRNAs. Also predicted and known targets of the microRNAs can be found on this site.

The term siRNA (short interfering RNA) as used herein has the same meaning as typically in the art. I.e. the term siRNA refers to double stranded RNA complex wherein the strands are typically 18-22 nucleotides in length. Very often, the complex has 3'-overhangs.

When referring to the RNAi machinery herein, what is meant are the cellular components necessary for the activity of siRNAs and/or microRNAs or for the RNAi pathway. A major player of the RNAi machinery is the RNA induced silencing complex (the RISC complex).

As referred to herein, an RNA unit is one of the monomers that make up an RNA polymer/oligomer. Thus, an RNA unit is also referred to as an RNA monomer or a RNA nucleotide. Likewise, a DNA unit is one of the monomers that make up a DNA polymer/oligomer and a DNA unit may also be referred to as a DNA monomer or a DNA nucleotide.

When referring to a base, what is meant is the base (also termed nucleobase) of a nucleotide. The base may be part of DNA, RNA, INA, LNA or any other nucleic acid capable of engaging in Watson Crick duplex formation and preferably in specific base pairing. The base may also be part of PNA (peptide nucleic acid) or morpholino. In some embodiments, the base may be a universal base.

When referring to the length of a sequence or oligonucleotide, reference may be made to the number of (repeating) units or to the number of bases.

When referring to a complementary sequence, G pairs to C, A pairs to T and U and vice versa. In a preferred embodiment, G also pairs to U and vice versa to form a so-called wobble base pair. In another preferred embodiment, the base inosine (I) may be substituted for A in any of SEQ ID NOs 1-723 (as may occur by A to I editing) or I may be substituted for A in sequences complementary to any of SEQ ID NOs 1-723. I basepairs to A, C and U. I may also be used in the molecules of the invention. In still another preferred embodiment, universal bases may be used in the molecules of the invention, e.g. no more than 1, 2 or 3 universal bases per molecule. Universal bases can typically basepair to G, C, A, U and T. Often universal bases do not form hydrogen bonds with the opposing base on the other strand. In still another preferred embodiment, a complementary sequence refers to a contiguous sequence exclusively of Watson-Crick base pairs. In the broadest aspect, a complementary sequence is a sequence that forms a duplex without mismatches.

The term complementary sequence has been defined above. The phrase "are capable of base pairing to" is related to the term complementary sequence. I.e. a first sequence is capable of base pairing to a second sequence, which is complementary to the first sequence.

A contiguous stretch of bases is intended to mean a non-interrupted sequence of bases that all fit into a duplex formed between the oligonucleotide and the target RNA. I.e. there are preferably no bulges in the duplex and it is preferred that the sequences are complementary (see the definition of complementary sequences above). Most preferred is perfect Watson-Crick duplex between the oligonucleotide of the invention and target region of the target RNA.

The terms contiguous and continuous are used interchangeably herein.

### Summary of the invention

The present invention provides bivalent molecules comprising a first oligonucleotide linked to a second oligonucleotide
- wherein the length of the first and the second oligonucleotide is between 7 and 12 nucleotides and at least 50% of the nucleotides of the first and the second oligonucleotide is locked nucleic acid (LNA) monomers
- wherein the first and the second oligonucleotide comprise an antisense sequence complementary to a cellular mRNA or microRNA and the antisense sequences act as steric blockers
- wherein the first oligonucleotide and the second oligonucleotide comprise
   i. a seed sequence of microRNA or
   ii. a sequence capable of base pairing to the complementary sequence of a seed sequence or
   iii. a sequence capable of base pairing to a seed sequence and
- wherein the first and second oligonucleotide is linked via a linking moiety with a length of at least 10 angstrom;
- wherein the linking moiety comprise a non-nucleotide polymer selected from the group consisting of: polyalkylen oxide, polyethyleneglycol , polyacrylic acid, polylactide acid (PLA), poly(glycolic acid) (PGA), polypropylene, polystyrene, polyolefin, polyamide, polycyanoacrylate, polyimide, polyethyleneterephtalat (PEY, PETG), polyethylene terephtalate (PETE), polytetramethylene glycol (PTG) and polyurethane; and
- wherein the first and the second oligonucleotide is not an aptamer, siRNA, ribozyme, RNase H activating antisense oligonucleotide, full unmodified RNA oligonucleotide or full unmodified DNA oligonucleotide and is incapable of recruiting the RNAi machinery and incapable of activating RNase H.

The bivalent molecules of the invention are useful for modulating microRNA regulatory pathways and may be used e.g. in research and as therapeutics. They may be used to block microRNA activity by binding to microRNA to thereby deregulate all targets of the microRNA. Importantly, the bivalent molecules of the invention may bind to (and inhibit or inactivate) two identical microRNAs (or microRNA families) or to two different microRNAs (or microRNA families).

They may also bind to microRNA binding site(s) in a target RNA to thereby prevent microRNA binding to the given microRNA binding site. This will prevent microRNA regulation of only the blocked target RNA, while other target RNAs of the microRNA can be left unaffected by the bivalent molecule.

In yet another embodiment, the first oligonucleotide of the molecule may bind a microRNA and the other oligonucleotide of the molecule may bind a microRNA binding site. In this way, a microRNA may be tethered to a mRNA via the bivalent molecule to impose microRNA regulation of the given mRNA.

### Detailed description

### Bivalent molecule

The present invention is directed to a bivalent molecule comprising a first oligonucleotide linked to a second oligonucleotide as defined in claim 1. The first oligonucleotide and/or the second oligonucleotide is not any of or is not selected from the group consisting of an aptamer, siRNA, ribozyme, RNase H activating antisense oligonucleotide, full unmodified RNA oligonucleotide or full unmodified DNA oligonucleotide and that the antisense oligonucleotides of the molecules of the invention are not capable of recruiting RNase H and/or RISC (the RNAi machinery).

Instead, the first and/or the second oligonucleotide comprise an antisense sequence complementary to a cellular RNA such as mRNA or microRNA and that the antisense sequences act as simple steric blockers.

The antisense sequence may be a Blockmir antisense sequence capable of binding to a microRNA binding site in a target RNA. The antisense sequence may also be an antimir antisense sequence capable of binding to a microRNA.

It is preferred that the first oligonucleotide and/or second oligonucleotide comprise a seed sequence (or a part of a seed sequence) of a microRNA, a sequence capable of base pairing to the complementary sequence of a seed sequence or a sequence capable of base pairing to a seed sequence.

Preferred microRNAs are human microRNAs and preferred mRNAs are also human.

### Sequences defined by complementarity

In a preferred embodiment, the first oligonucleotide and/or the second oligonucleotide of the molecule of the invention comprise
a. A contiguous sequence of at least 5 nucleotides that is capable of base pairing to the complementary sequence of one of SEQ ID NOs 1-723 (Blockmir antisense sequence) or
b. A contiguous sequence of at least 5 nucleotides that is capable of base pairing to one of SEQ ID NOs 1-723 (antimir antisense sequence)

- Wherein 1, 2, or 3 A's in any of SEQ ID NOs 1-723 may be substituted with I (inosine) and wherein I base pairs to A, C and U and wherein wobble G-U base pairs are allowed, alternatively
- Wherein 1, 2, or 3 A in the SEQ ID NO may not be substituted with I and wherein wobble G-U base pairs are allowed, alternatively
- wherein 1, 2, or 3 A in the SEQ ID NO may not be substituted with I and wherein wobble G-U base pairs are not allowed.

As will be recognized, "*a contiguous sequence of at least 5 nucleotides that is capable of base pairing to the complementary sequence of one of SEQ ID NOs:1-723"* is a sequence that may bind to the same sequence as a microRNA (represented by a given SEQ ID NO). Such sequences may herein be referred to as Blockmir antisense sequences or just Blockmir sequences.

As will be recognized, *"A contiguous sequence of at least 5 nucleotides that is capable of base pairing one of SEQ ID NOs 1-723"* is a sequence that may bind to a microRNA (represented by a given SEQ ID NO). Such sequences may herein be referred to as antimir antisense sequences or just antimir sequences.

The contiguous sequences (antimir or Blockmir as described above) are of between 7 and 12 nucleotides.

As mentioned, in one embodiment, both the first and the second oligonucleotide comprise a Blockmir antisense sequence. In another embodiment, both the first and the second oligonucleotide comprise an antimir antisense sequence. And in yet another embodiment, the first oligonucleotide comprises a Blockmir antisense oligonucleotide and the second oligonucleotide comprises an antimir antisense oligonucleotide.

### Very short fully modified oligonucleotides

One advantage of the present invention is that it enables the use of very short oligonucleotides, because the first and the second oligonucleotide will bind cooperatively to their target RNAs.

When both the first and the second oligonucleotide binds to the same target RNA (same entity), the binding energy for each oligonucleotide can be added (giving an exponential increase in binding affinity) and hence it may be said that the oligonucleotides will bind cooperatively (the first oligonucleotide significantly increases the binding affinity of the second oligonucleotide and vice versa). It should be recognized though that the term cooperative may be misleading in this context because the first and the second oligonucleotide is part of the same molecule. However, if the first and the second oligonucleotide are regarded as separate entities, it is clear that they will bind cooperatively. Moreover, if the first and the second oligonucleotide are tested individually in terms of binding to a target RNA, they will have much reduced affinity as compared to the bivalent counterpart and most often, they will also have reduced activity.

When the first and the second oligonucleotide binds to two separate microRNAs (identical or different), cooperativity is expected because microRNAs in general bind cooperatively to target RNAs. I.e. a first microRNA bound to a given target RNA typically facilitates binding of a second microRNA to the same target RNA. Not intended to be bound by theory, it is believed that a first and second microRNA bound the same target RNA often interacts to create additional binding energy and hence cooperative binding.

Thus, if the first and the second oligonucleotide are tested individually in terms of binding to a target RNA, they will have much reduced affinity as compared to the bivalent counterpart and most often, they will also have reduced activity if they have any activity at all.

In addition to the advantages regarding binding affinities, the molecules of the invention also have other specific advantages e.g. relating to biodistribution in the organism as well as within organs and single cells. This particular applies for the use of a very short first and/or second oligonucleotide. Moreover, advantages in terms of duration of action may be observed, possibly caused by improved biostability.

If the oligonucleotides are 15 or shorter, they may be fully modified with affinity increasing nucleotide analogues (e.g. LNA or other 2'-O-modifications). This becomes increasingly relevant with decreasing length.

Thus, in a preferred embodiment, the bivalent molecules of the invention may comprise a first oligonucleotide of e.g. 8 nucleotides (e.g. LNA or other 2'-O-modified nucleotides) complementary to position 2-9 of a first microRNA and a second oligonucleotide of e.g. 8 nucleotides (e.g. LNA or other 2'-O-modified nucleotides) complementary to position 2-9 of a second microRNA. The first and the second microRNA may be the same or they may be different. Importantly, when using very short antisense sequences, microRNA families sharing the same seed sequence may be targeted. I.e. the molecules of the invention enable targeting of two different microRNAs or two different microRNA families with the same molecule. This cannot be achieved using the molecules currently part of the state of the art, in particular not exogenously synthesized molecules comprising less than 30 or 20 nucleotides.

In another preferred embodiment, the first oligonucleotide may consist of a Blockmir antisense sequence of a length of 7-9 nucleotides (e.g. LNA or other 2'-O-modified nucleotides, specific sequences are given above) comprising the seed sequence of a first microRNA and second oligonucleotide may consist of a Blockmir antisense sequence of a length of 7-9 nucleotides (e.g. LNA or other 2'-O-modified nucleotides) comprising the seed sequence of a second microRNA, wherein the first and the second microRNA may be different or identical. Thus, if a given microRNA has two binding sites in the same target RNA, both binding sites may both be blocked using the same bivalent molecule. Likewise if the same target RNA is regulated by two different microRNAs, both microRNA binding sites may be blocked by the same bivalent molecule. This cannot be achieved using the molecules currently part of the state of the art, in particular not exogenously synthesized molecules comprising less than 30 or 20 nucleotides

### Activity of oligonucleotides

As mentioned above the first and the second oligonucleotide do not recruit the RNAi machinery or RNase H. Likewise, the oligonucleotides should not act as a ribozyme, DNAzyme or aptamer.

The oligonucleotides are steric blockers. This can be achieved by a modification pattern that makes the oligonucleotide incompatible with RNase H and the RNAi machinery as is further described below.

### RNase H cleavage

RNase H cleaves the RNA part of a RNA-DNA duplex. The structural requirements for RNase H activation are well-known to the skilled man. This mechanism is very often used to achieve traditional antisense regulation e.g. by employing so-called gapmers. Gapmers are antisense oligonucleotides that comprise a central region with deoxy sugars (the gap) and modified flanks. Gapmers very often comprises phosphorothioate internucleotide linkages to improve biostability and the flanks comprise e.g. 2-O-modifications that also improve biostability, i.e. resistance against nucleolytic attack and increase the melting temperature of the gapmer base paired to a complementary nucleic acid. Also headmer and endmer structures have been described in the literature.

As mentioned that the oligonucleotide of the molecules of the invention is not capable of inducing RNase H cleavage of the target RNA. The skilled man is well aware of the requirements for RNase H cleavage and will be able to design oligonucleotides that do or do not activate RNase H. The skilled man will also be capable of testing whether oligonucleotides do or do not activate RNase H. Most importantly, the oligonucleotide should not contain extended stretches of unmodified DNA.

Thus, it is preferred that the oligonucleotide does not comprise a stretch of unmodified DNA that exceeds a length selected from the group consisting of: 3 bases, 4 bases, 5 bases, 6 bases, 7 bases, 8 bases, 9 bases, 10 bases and 11 bases. Most preferably, the stretch of unmodified DNA does not exceed 3 bases. In another preferred embodiment, the oligonucleotide does not comprise any DNA monomers.

A positive description of all allowed modifications that will prevent RNase H activation is not feasible, since a very wide variety of modifications will do that. Particular preferred modifications and patterns are described below.

### RNAi machinery

The RNAi machinery is a sophisticated gene regulatory system that is guided by RNA. Thus, microRNAs guide the RNAi machinery to target mRNAs to affect the activity of the target mRNA. The RNAi machinery may affect translation of the mRNA directly or it may affect the stability of the target mRNA, i.e. mediate direct degradation of the target mRNA. Not intended to be bound by theory, it is believed that the degree of complementarity between microRNA and target mRNA is a key element as to whether the target mRNA is subjected to translational regulation or degradation.

Endogenous microRNAs are processed from precursor stem-loops and incorporated into a so called RNA induced silencing complex (RISC complex). The details of this process are still poorly understood.

The cellular RNAi machinery has been extensively used to affect the activity of cellular mRNAs by introducing synthetic double stranded RNA complexes termed siRNAs into the cell. As mentioned above, siRNAs are short double stranded RNA complexes comprising a passenger strand and a complementary guide strand. The guide strand of siRNA is incorporated into the RISC complex, where after the RISC complex can affect the activity of mRNA harbouring complementary sequences to the guide strand. Thus, siRNAs are a new class of compounds that is thought to be capable of efficiently and specifically targeting any mRNA and consequently, siRNAs are regarded potentially as a new class of therapeutics.

A common feature of siRNAs and microRNAs is that they recruit the cellular RNAi machinery to affect the activity of target RNAs.

As mentioned, the oligonucleotides of the molecules of the invention are not capable of recruiting the RNAi machinery and hence direct the RNAi machinery to the target RNA. I.e. the oligonucleotides of the molecules of the invention should not be designed as siRNA or microRNA.

The skilled man is well aware of the requirements for recruitment of the RNAi machinery and will be able to design oligonucleotides that do or do not recruit the RNAi machinery. Moreover, the skilled man will be capable of testing whether oligonucleotides do or do not recruit the RNAi machinery.

It is particular preferred that the oligonucleotides are single stranded and that they are not fully RNA - as opposed to a siRNA designed for recruiting the RNAi machinery.

In one embodiment the oligonucleotide does not comprise a stretch of unmodified RNA monomers that exceeds a length selected from the group consisting of: 3 bases, 4 bases, 5 bases, 6 bases, 7 bases, 8 bases, 9 bases, 10 bases and 11 bases. Most preferably, the stretch of unmodified RNA does not exceed 3 bases. This will ensure that the oligonucleotide does not recruit the RNAi machinery. However, it must be recognized that in some embodiments, the oligonucleotide can indeed comprise more than 3 contiguous RNA units. In such embodiment, heavy modification of the rest of the oligonucleotide may be used to prevent recruitment of the RNAi machinery.

In another preferred embodiment, the oligonucleotide does not comprise any RNA monomers.

A positive description of all allowed modifications that will prevent recruitment of the RNAi machinery is not feasible, since a very wide variety of modifications will do that. Particular preferred modifications and patterns are described below.

### Chemistry and architecture

As described the oligonucleotides of the molecules of the invention do not recruit the RNAi machinery and at the same time do not recruit RNase H. Moreover, it is desired that the oligonucleotides have a sufficient bioavailability and stability. These characteristics can be achieved by appropriate chemical modifications.

Since only very specific oligonucleotide architectures and chemistry allow recruitment of RNase H and the RNAi machinery, the oligonucleotides of the molecules of the invention are best described by way of non-allowed structures or negative limitations as described above.

Hereafter, a number of allowed and preferred modifications are described. Again it is emphasized that it is impossible to exhaustively describe all allowed modifications which will enable the oligonucleotides to act as steric blockers. In general it may be said that this can be achieved by a modification pattern that makes the oligonucleotide incompatible with RNase H and the RNAi machinery.

### Nucleotide analogues and modifications

As mentioned the first and/or second oligonucleotide comprise nucleotide analogues such as to improve affinity, bioavailability and biostability and both oligonucleotides prevent recruitment of the RNAi machinery and RNase H activation.

The fraction of units modified (LNA) relatively to the units not modified is more than 50 %.

Further, in a preferred embodiment, phosphorothioate internucleotide linkages may connect the units to improve the biostability of the oligonucleotide. Thus, the first and/or the second oligonucleotide may be fully phosphorothiolated or only partly phosphorothiolated.

In another embodiment, the fraction of phosphorothioate linkages is selected from the group consisting of 100%, less than 95%, less than 90%, less than 85 % or less than 75%, less than 70%, less than 65%, less than 60%, less than 50 %, more than 95%, more than 90%, more than 85 % or more than 75%, more than 70%, more than 65%, more than 60% and more than 50 %.

The oligonucleotide may also comprise phosporoamidate linkages, and preferably fraction of phosporoamidate linkages is linkages is selected from the group consisting of 100%, less than 95%, less than 90%, less than 85 % or less than 75%, less than 70%, less than 65%, less than 60%, less than 50 %, more than 95%, more than 90%, more than 85 % or more than 75%, more than 70%, more than 65%, more than 60% and more than 50 %.

As should be clear the modifications and nucleotide analogues may be combined and it should be clear that phosphoroamidate and phosphorothioate modifications can be used in combination with sugar or base modifications at the same unit position. Thus, LNA phosphoromidates may e.g. used.

In a preferred embodiment, the oligonucleotide comprises a repeating pattern of LNA units and one or more units that are substituted in the 2'-position. OMe/LNA mixmers have been shown to be powerful reagents for use as steric block inhibitors of gene expression regulated by protein-RNA interactions. Thus, when the oligonucleotides of the invention are used to block the activity of a microRNA at a target RNA, a OMe/LNA mixmer architecture (preferably connected by phosphorothioate linkages) may be used. A gapmer structure may also be used, however without being capable of inducing RNase H if the oligonucleotide is intended to act as a Blockmir.

In another preferred embodiment, the oligonucleotide comprises exclusively LNA units and DNA units and these may be connected by phosphorothioate linkages as outlined above.

In still another embodiment, the first and/or the second oligonucleotide of the invention does not comprise any morpholino units and/or PNA units and/or 2'-O-modified RNA units and/or unmodified DNA units and/or unmodified RNA units. When reference is made to unmodified DNA and RNA, the interlinkage may (or may not) be e.g. phosphorothioate or phoshoroamidate.

### Linking moiety

The first and second oligonucleotide is linked via a linking moiety.

The linking moiety may be attached to the nucleobase or to the sugar phosphate backbone of the first and second oligonucleotide.

The linking moiety may e.g. be a polypeptide, polysaccharide, C8, C6, or C12.

The linking moiety consist of or comprise a non-nucleotide polymer such as polyalkylen oxide, polyethyleneglcyol for example alpha-, omega-dihydroxypolyethylenglycol, biodegradable lactone-based polymers e.g. polyacrylic acid, polylactide acid (PLA), poly(glycolic acid) (PGA), polypropylene, polystyrene, polyolefin, polyamide, polycyanoacrylate, polyimide, polyethyleneterephtalat (PEY, PETG), polyethylene terephtalate(PETE), polytetramethylene glycol (PTG), polyurethane (as well as mixtures thereof). Most preferred is polyalkylene glycol such as polyethylene glycol.

Pegylation of oligonucleotides and methods for preparation of pegylated oligonucleotides have e.g. been described in Nucleic Acids Research, 1994, Vol. 22, No. 22, 4810-4817, WO2008/077956 and WO2005/111238.

In a preferred embodiment, the linking moiety is attached during oligonucleotide synthesis such that when the first oligonucleotide have been synthesized, the linking moiety is attached to the first oligonucleotide, where after the second oligonucleotide is synthesized. I.e. the linking moiety adapted for use in standard oligonucleotide synthesis may be used.

Examples of commercially available linking moieties adapted for incorporation into an oligonucleotide are:
Spacer 18 amidite (17-O-DMT-Hexaethyleneoxide-1-O-phosphoramidite), Spacer 9 Amidite (8-DMT-O-Triethyleneoxide-1-O-phosphoramidite), C6 Spacer Amidite (6-DMT-O-Hexanediol-1-O-Phosphoramidite) and C3 Spacer Amidite (DMT-1,3 propanediol-phosphoramidite). As will be recognized, multiples of linking moieties may be incorporated to obtain a desired linker length, e.g. between 1 and 100, such as between 1 and 50, between 1 and 25, between 1 and 10, between 1 and 9, between 1 and 8, between 1 and 7, between 1 and 6, between 1 and 5, between 1 and 4, between 1 and 3, and between 1 and 2.

The length of the linking moiety may be adjusted according the specific use of the particular bivalent molecule. If the first and the second oligonucleotide of the molecule comprise Blockmir antisense sequences, the length of the linking moiety may be adjusted according to the distance between the two microRNA binding sites in the target RNA. Thus, if the binding sites are separated by 20 nucleotides, then the linking moiety may have a length between 10 and 70 angstrom based on the distance between nucleotides in a linear nucleic acid. Normally, there should be no reason to use a linker that is significantly longer than the linear distance between binding sites. On the other hand, it should be recognized that the sites may be much closer than the linear distance because of the three dimensional structure of the target RNA. Therefore, it is typically recommended to test various linking moieties with varying lengths. It is generally preferred that the linking moiety is no more than 1000 angstrom in length, such as no more than 900, 800, 700, 600, 500, 400, 300, 200 or 100 angstrom in length. The linking moiety is at least 10 angstrom in length, such as 15, 20, 25, 30, 35 or 40 angstrom in length.

Preferred ranges are between 10 and 1000 angstrom, between 20 and 500 angstrom and between 20 and 200 angstrom.

When the first and the second oligonucleotide both comprise antimir antisense sequences, the linking moiety will typically have a length between 10 and 100 angstrom, more preferably between 20 and 75 angstrom.

### Delivery

Various methods for delivery of oligonucleotides are known to the skilled man. Thus, oligonucleotides may be formulated in microparticles and nanoparticles. Liposomes are frequently used as delivery vehicle and a variety of liposome delivery systems exist. They may e.g. comprise cationic lipids or neutral lipids. Their size may be varied for various purposes and other components may be included in the liposomes or on the surface of the liposomes. Chitosan nanoparticles have been used for delivery of plasmids and siRNAs to various cells, among them primary cells. Thus, chitosan nanoparticles may also be used for delivery of the oligonucleotides of the invention. Others polymers for delivery are polyethyleneimine (PEI), cyclodextrin, atelocollagen, polyamidoamine (PAMAM) and poly(lactic-co-glycolic acid) (PLGA). Further, oligonucleotides of the invention may be conjugated to cationic peptides that have been shown to facilitate transport into cells. The oligonucleotides may also be conjugated to lipids to facilitate delivery. In particular, cholesterol conjugation has been used to improve antimir delivery.

A second aspect of the invention is the use of the molecule of the invention for modulating microRNA regulation either by blocking a microRNA or by blocking a microRNA binding site in a target RNA, either in vivo or in vitro.

A third aspect of the invention is the molecule of the invention for use in therapy, e.g. treatment of HCV infection.

### Examples

### Example 1

### Bivalent molecules for treatment of HCV.

### Blockmirs:

### Background

It has been demonstrated that mir-122 modulates Hepatitis C virus RNA abundance by facilitating replication of the viral RNA (Jopling CL, 2005). The 5'UTR of the HCV genom comprises two conserved antiseed sequence capable of base pairing with the seed sequence of microRNA-122.

It has been demonstrated that the level of HCV viral replicon RNA was reduced by app. 80 % when mir-122 was inactivated by a so-called antagomir.

A genetic interaction between mir-122 and the 5' noncoding region of the viral genom was revealed by mutational analysis of the predicted micro RNA binding site and ectopic expression of mir-122 molecules containing compensatory mutations.

### Bivalent antimirs targeting microRNA-122

A described, antimir inactivation of microRNA-122 has been demonstrated and microRNA-122 inactivation affects HCV replication. As an alternative, bivalent molecules comprising a first and/or a second antisense sequence directed to microRNA-122 may be employed. Such bivalent molecules may be more potent that than monovalent antimirs because two microRNAs will bind cooperatively to the bivalent molecule. Moreover, they may also have a more favourable biodistribution, because the bivalent molecules in some aspects may have the characteristics of the overall size of the molecule, while in other aspects, the bivalent molecules may have characteristics of the smaller (first and second) oligonucleotides of the bivalent molecule. This may e.g. be the case with respect to entry into cells.

The sequence of mir-122 with the seed sequence underlined is:
3'UGUUUGUGGUAACAG**UGUGAGG**U

Base paired to a complementary sequence:
3'UGUUUGUGGUAACAG**UGUGAGG**U
5'ACAAACACCATTGTC**ACACTCC**A

Three bivalent molecules targeting microRNA-122 may e.g. be:
a) TC**ACACTCC-**----TC**ACACTCC**
b) C**ACACTCC**-----C**ACACTCC**
c) TC**ACACTCC**-----C**ACACTCC**

Wherein ----- denote a linker, e.g. a PEG linker.
The oligonucleotides may e.g. consist entirely of LNA monomers.

More specific embodiments are described in the detailed description.

### Bivalent Blockmirs targeting HCV

The sequence of the target region (anti-seed sequence is bold) in the 5'noncoding region is:
CCAGCCCCCTGATGGGGGCG**ACACTCCA**CCATGAAT**CACTCC**CCTGTGAGGAACTACTGT

And with the complementary sequence indicated:
5'CCAGCCCCCTGATGGGGGCG**ACACTCCA**CCATGAAT**CACTCC**CCTGTGAGGAACTACT
3'GGTCGGGGGACTACCCCCGC**TGTGAGGT**GGTACTTA**GTGAGG**GGACACTCCTTGATGA

Bivalent molecules may e.g. be:

5′CCAGCCCCCTGATGGGGGCGACACTCCACCATGAATCACTCCCCTGTGAGGAACTACT
GCTGTGAGGT-------AGTGAGG5′

5′CCAGCCCCCTGATGGGGGCGACACTCCACCATGAATCACTCCCCTGTGAGGAACTACT
TGTGAGGT------TAGTGAGG5′

Wherein ----- denote a linker, e.g. a PEG linker.
The oligonucleotides may e.g. consist entirely of LNA monomers.

More specific embodiments are described in the detailed description.

### Example 2

### Bivalent molecules for treatment of cancer

MicroRNA-21 plays is upregulated in various cancers and therefore there is interest in down regulation of microRNA-21.

The sequence of microRNA-21 is:
3'AGUUGUAGUCAGAC**UAUUCGA**U

Basepaired to a complementary sequence:
3'AGUUGUAGUCAGAC**UAUUCGA**U
5'TCAACATCAGTCTG**ATAAGCT**A:

Four bivalent molecules targeting microRNA-122 may e.g. be:
a) TG**ATAAGCT**-----TG**ATAAGCT**
b) G**ATAAGCT**-----G**ATAAGCT**
c) **ATAAGCT-----ATAAGCT**
d) TG**ATAAGCT**----- **ATAAGCT**

Wherein ----- denote a linker, e.g. a PEG linker.
The oligonucleotides may e.g. consist entirely of LNA.

More specific embodiments are described in the detailed description.

### Example 3

Bivalent molecules useful for treatment of psoriasis.
It has been demonstrated that psoriasis is characterized by a specific miRNA expression profile that differs from that of healthy skin or another chronic inflammatory disease, atopic eczema. Among miRNAs overexpressed in psoriasis, a keratino cytespecific miRNA (miR-203) and a leukocyte-derived miRNA (miR-146a) were identified.

The up-regulation of miR-203 in psoriatic plaques was concurrent with the down-regulation of an evolutionary conserved target of miR-203, suppressor of cytokine signaling 3 (SOCS-3), which is involved in inflammatory responses and keratinocytefunctions (Sonkoly E, 2007, Jul 11).

Another study showed that miR-146a, one of the psoriasis-specific miRNAs, inhibits the expression of IRAK-1 (interleukin-1 receptor-associated kinase 1) and TRAF-6 (TNF receptor-associated factor 6) proteins both of which are regulators of the TNF-a signalling pathway (Taganov KD, 2006). Hence, it is conceivable that miR-146a is involved in the pathogenesis of psoriasis via the modulation of TNF-a signalling in the skin.

One bivalent molecule can inactivate microRNA-146 and microRNA-203. The sequences of the microRNAs are:
Mir-203:
   3'GAUCACCAGGAUUU**GUAAAGU**G

Base paired to a complementary sequence:
3'GAUCACCAGGAUUU**GUAAAGU**G
5'CTAGTGGTCCTAAA**CATTTCA**C

Mir-146:
   3' UUGGGUACCUUAAG**UCAAGAG**U

Base paired to a complementary sequence:
3'UUGGGUACCUUAAG**UCAAGAG**U
5'AACCCATGGAATTC**AGTTCTC**A

Exemplary bivalent molecules targeting microRNA-203 and microRNA-146a:
a) AA**CATTTCA**-----TC**AGTTCTC**
b) A**CATTTCA**-----C**AGTTCTC**
c) **CATTTCA-----AGTTCTC**
d) AA**CATTTCA-----AGTTCTC**
e) TC**AGTTCTC**-----AA**CATTTCA**
f) C**AGTTCTC**-----A**CATTTCA**
g) **AGTTCTC-----CATTTCA**
h) TC**AGTTCTC-----CATTTCA**

Wherein ----- denote a linker, e.g. a PEG linker.
The oligonucleotides may e.g. consist entirely of LNA.

More specific embodiments are described in the detailed description.

### Example 4

To test the activity of various bivalent oligonucleotides, a reporter gene construct was made wherein a hepatitis C sequence comprising two microRNA-122 binding sites was cloned behind the renilla luciferase gene in the psiCHECK™-2 Vector. When this plasmid is transfected into cells expressing microRNA-122, or co-transfected with microRNA-122, the microRNA will bind to the binding sites and repress expression of the reporter gene. I.e. the activity of bivalent molecules of the invention targeted to microRNA-122 or the microRNA-122 bindingssites in the reporter construct can easily be tested.

The vector construct was made using the following two oligonucleotides:
HCV-Downstream:
   **GCCA**GCGGCCGGC**GGGGAGTGATTCATGGTGGAGTGTCGCCCC**
HCV-Upstream:
   **ATCG**CTCGAG**GCCAGCCCCCTGATGGGGGCGACACTCCAC**
These were annealed and extended in a PCR reaction, where after the double stranded DNA was digested with XhoI and Not-I and cloned into the XhoI and NotI sites of the psiCHECK™-2 Vector.

### Bivalent oligonucleotides tested:

(030) ANTIMIR122 CONTROL
   5'-LCMCLAMTMTLGLTMCMALCMALCMTLCLC-3'
   Antimir control targeting microRNA-122.
(034) HCV Fullmatch 5'-
   LGLGMALGMULGMALTMULCMAMULGMGMULGMGLALGMUMGLTLC-3'
   Bivalent Blockmir targeted to HCV RNA and which is expected to mask both microRNA-122 binding sites. The linking moiety consists of nucleotides.
(035) All targets full LNA 8-mer 5'-LTLGLGLALGLTLGLT-3'
   Monovalent Blockmir with perfect complementarity to target 1 and incomplete complementarity to target 2. See alignment below.
(037) HCV T1 LINK20 T2
   5'-LGLGLGLALGLTLGLA3' -X- 5'LTLGLGLALGLTLGLT-3'
   Bivalent Blockmir targeted to HCV RNA and which is expected to mask both microRNA-122 binding sites. The linking moiety is a PEG linker, see structure below.
(038) HCV T1 LINK40 T2
   5'- LGLGLGLALGLTLGLA3' -XX- 5'LTLGLGLALGLTLGLT-3'
   Bivalent Blockmir targeted to HCV RNA and which is expected to mask both microRNA-122 binding sites. The linking moiety is a PEG linker, see structure below.
(043) Bivalent antimir 21a (linker 20)
   5'-LGLALTLALALGLCLT3'-X-5'LGLALTLALALGLCLT-3'
   Bivalent antimir targeted to microRNA-21.
(056) MIR122 BIVALENT 20:
   5' LCLALCLALCLTLCLC3'- X-5'LCLALCLALCLTLCLC3'
   Bivalent antimir targeted to microRNA-122.
(057) MIR122 BIVALENT 40: 5'LCLALCLALCLTLCLC3'-XX-5'LCLALCLALCLTLCLC3' Bivalent antimir targeted to microRNA-122.
   L denote a LNA nucleotide
   M denote a 2'O-methyl-RNA nucleotide
   X denote a linker: 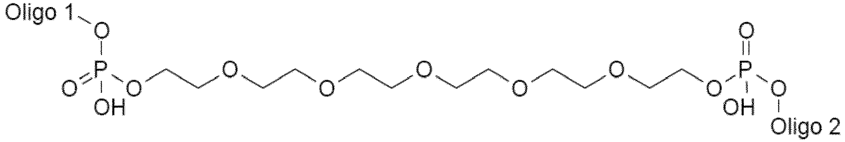 X is incorporated during standard oligonucleotide synthesis using a phosporoamidite:

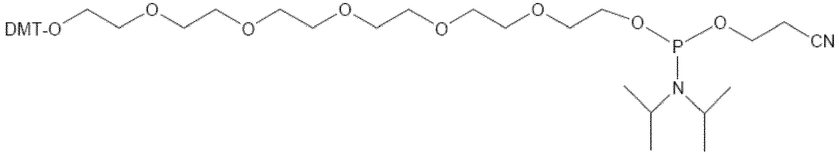

To illustrate where the Blockmirs bind to the HCV targets, the reverse complements of the Blockmirs are here shown aligned with the HCV target sequences.

34 GACACTCCACCATGAATCACTCC
35 ACACTCCA ACACTCCA
37 ACACTCCA---X---ACACTCCC
38 ACACTCCA---XX--ACACTCCC
1A: GCCAGCCCCCTGATGGGGGCGACACTCCACCATGAATCACTCCCCTGTGAGGAACTACTGT
binding site 1 binding site 2

### Results

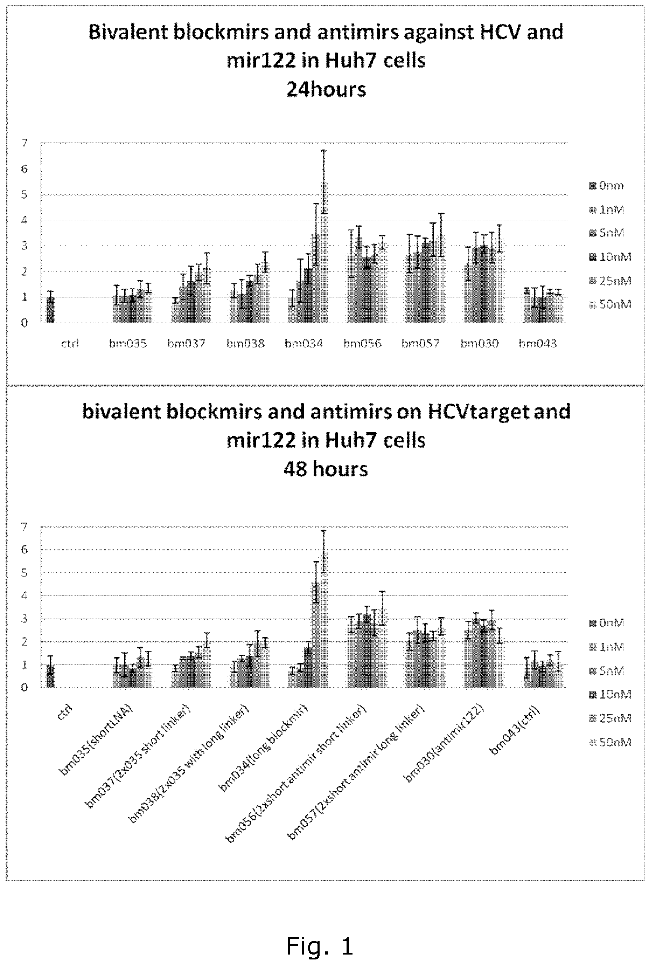

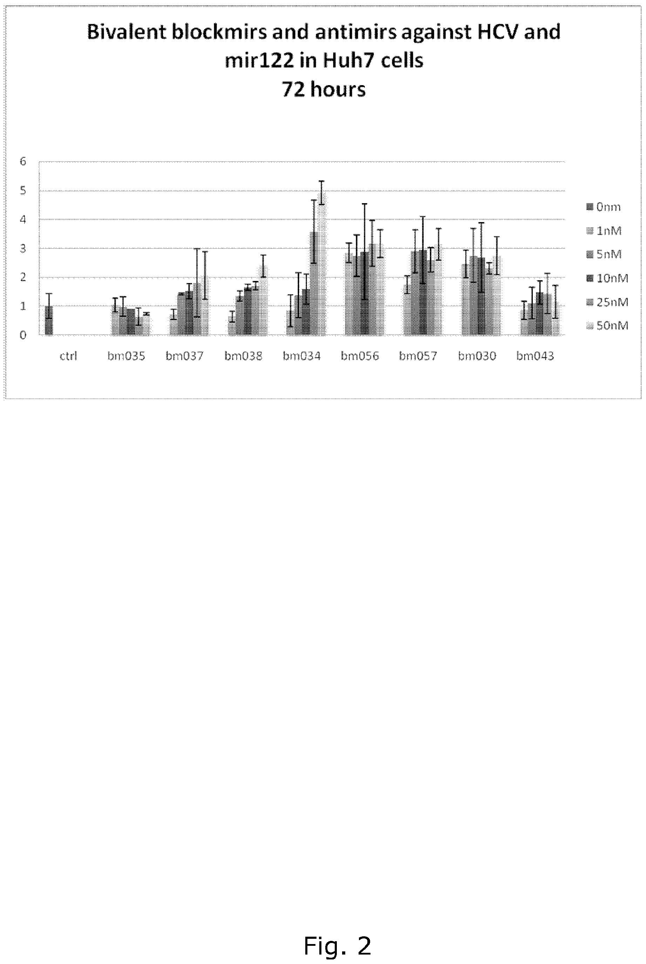

The oligonucleotides and the reporter plasmid was transfected into HUH7 cells expressing microRNA-122 using lipofectamin 2000. Dual luciferase activity (renilla luc vs firefly luc) was measured after 24, 48 and 72 hours. The results are shown in figures 1 and 2. The control bar is mock transfected HUH7 cells, i.e. the control shows the repressed rluc expression. Another control is transfection of 43, which is a bivalent antimir targeted to microRNA-21.

As expected, when the cells have been transfected with a standard antimir (bm030) directed to microRNA-122, rluc is derepressed. When the cells are transfected with bivalent antimirs 56+57, rluc is derepressed with a similar potency as the reference antimir (bm030).

When the cells where transfected with bivalent Blockmirs 37, 38 and 34, in all cases rluc was derepressed, mostly so by Blockmir 34. Importantly, monovalent Blockmir 35 identical to one of the Blockmir antisense oligonucleotides of 34 and 35 had no effect of rluc expression. Thus, going from monovalent to bivalent molecules significantly increased potency.

### Example 5

Since the bivalent antimir molecules of example 4 had approximately the same potency as the reference antimir compound, the activities of the compounds were tested in lower concentrations. In addition, three new bivalent antimir molecules were tested.

### New bivalent oligonucleotides tested:

(120)
   LAMCMALCMULCLCMAMCMCMALTMGMAMAMULCMALCMULCLC
   Bivalent antimir targeted to microRNA-122. The linking moiety consists of nucleotides. The antiseed sequences are underlined.
(121)
   LGMCLCMAMAMCMALCMULCLCMAMCMCMAMUMGMAMAMULCMALCMUMCLC
   Bivalent antimir targeted to microRNA-122. The linking moiety consists of nucleotides. The antiseed sequences are underlined.
(122)
   LALCMALCMULCMCMAMCMCMALCMALCMUMCLC
   Bivalent antimir targeted to microRNA-122. The linking moiety consists of nucleotides. The antiseed sequences are underlined.
   L denote a LNA nucleotide
   M denote a 2'O-methyl-RNA nucleotide

### Results

The oligonucleotides were tested as outlined in example 4.

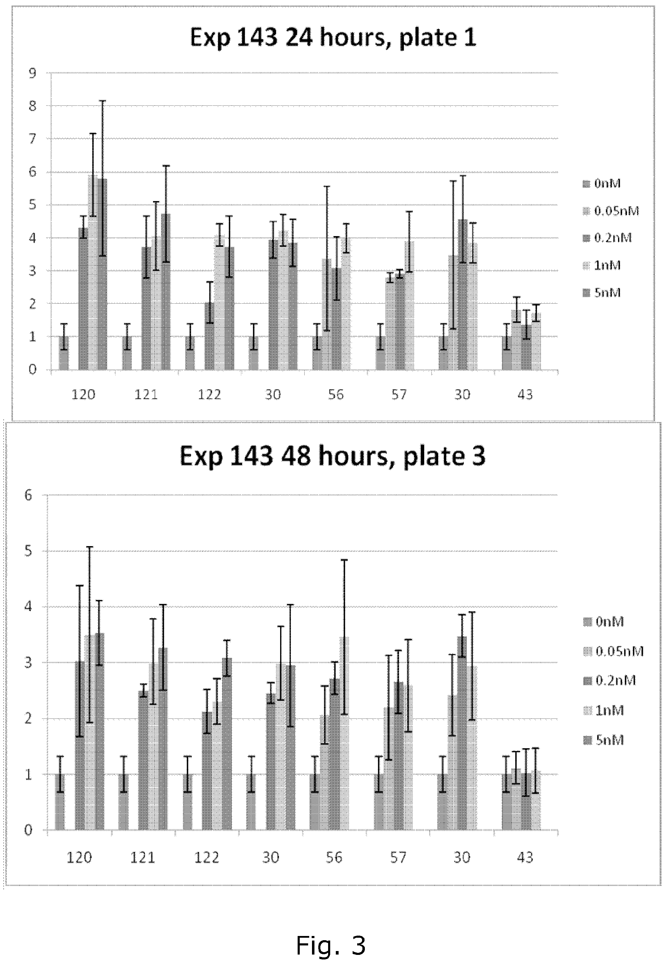

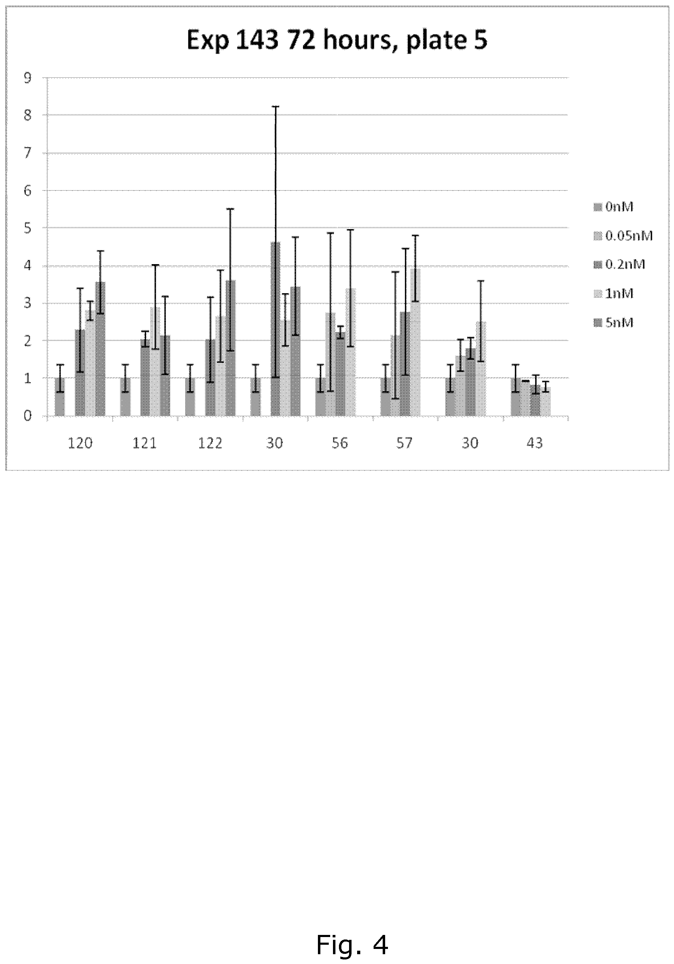

As shown in figure 3 and 4, even at 0,2 nM and 0,05 nM no significant difference in potency was observed between the reference antimir 30 and bivalent antimirs 56 and 57. I.e. the bivalent antimirs were very potent. Moreover, there was a slight tendency for bivalent antimirs 56 and 57 to have a longer duration of action as they appeared more potent than the reference antimir after 72 hours.

Also new bivalent antimirs 120, 121 and 122 had potency comparable to the reference antimir. Thus, bivalent antimirs with a linking moiety of nucleotides functions effectively.

**SEQUENCE LISTING**

| MicroRNA | Sequence | SEQ ID NO |
|---|---|---|
| hsa-let-7a | UGAGGUAGUAGGUUGUAUAGUU | 1 |
| hsa-let-7a* | CUAUACAAUCUACUGUCUUUC | 2 |
| hsa-let-7b | UGAGGUAGUAGGUUGUGUGGUU | 3 |
| hsa-let-7b* | CUAUACAACCUACUGCCUUCCC | 4 |
| hsa-let-7c | UGAGGUAGUAGGUUGUAUGGUU | 5 |
| hsa-let-7c* | UAGAGUUACACCCUGGGAGUUA | 6 |
| hsa-let-7d | AGAGGUAGUAGGUUGCAUAGUU | 7 |
| hsa-let-7d* | CUAUACGACCUGCUGCCUUUCU | 8 |
| hsa-let-7e | UGAGGUAGGAGGUUGUAUAGUU | 9 |
| hsa-let-7e* | CUAUACGGCCUCCUAGCUUUCC | 10 |
| hsa-let-7f | UGAGGUAGUAGAUUGUAUAGUU | 11 |
| hsa-let-7f-1* | CUAUACAAUCUAUUGCCUUCCC | 12 |
| hsa-let-7f-2* | CUAUACAGUCUACUGUCUUUCC | 13 |
| hsa-let-7q | UGAGGUAGUAGUUUGUACAGUU | 14 |
| hsa-let-7g* | CUGUACAGGCCACUGCCUUGC | 15 |
| hsa-let-7i | UGAGGUAGUAGUUUGUGCUGUU | 16 |
| hsa-let-7i* | CUGCGCAAGCUACUGCCUUGCU | 17 |
| hsa-miR-1 | UGGAAUGUAAAGAAGUAUGUAU | 18 |
| hsa-miR-100 | AACCCGUAGAUCCGAACUUGUG | 19 |
| hsa-miR-100* | CAAGCUUGUAUCUAUAGGUAUG | 20 |
| hsa-miR-101 | UACAGUACUGUGAUAACUGAA | 21 |
| hsa-miR-101* | CAGUUAUCACAGUGCUGAUGCU | 22 |
| hsa-miR-103 | AGCAGCAUUGUACAGGGCUAUGA | 23 |
| hsa-miR-105 | UCAAAUGCUCAGACUCCUGUGGU | 24 |
| hsa-miR-105* | ACGGAUGUUUGAGCAUGUGCUA | 25 |
| hsa-miR-106a | AAAAGUGCUUACAGUGCAGGUAG | 26 |
| hsa-miR-106a* | CUGCAAUGUAAGCACUUCUUAC | 27 |
| hsa-miR-106b | UAAAGUGCUGACAGUGCAGAU | 28 |
| hsa-miR-106b* | CCGCACUGUGGGUACUUGCUGC | 29 |
| hsa-miR-107 | AGCAGCAUUGUACAGGGCUAUCA | 30 |
| hsa-miR-10a | UACCCUGUAGAUCCGAAUUUGUG | 31 |
| hsa-miR-10a* | CAAAUUCGUAUCUAGGGGAAUA | 32 |
| hsa-miR-10b | UACCCUGUAGAACCGAAUUUGUG | 33 |
| hsa-miR-10b* | ACAGAUUCGAUUCUAGGGGAAU | 34 |
| hsa-miR-122 | UGGAGUGUGACAAUGGUGUUUG | 35 |
| hsa-miR-122* | AACGCCAUUAUCACACUAAAUA | 36 |
| hsa-miR-124 | UAAGGCACGCGGUGAAUGCC | 37 |
| hsa-miR-124* | CGUGUUCACAGCGGACCUUGAU | 38 |
| hsa-miR-125a-3p | ACAGGUGAGGUUCUUGGGAGCC | 39 |
| hsa-miR-125a-5p | UCCCUGAGACCCUUUAACCUGUGA | 40 |
| hsa-miR-125b | UCCCUGAGACCCUAACUUGUGA | 41 |
| hsa-miR-125b-1* | ACGGGUUAGGCUCUUGGGAGCU | 42 |
| hsa-miR-125b-2* | UCACAAGUCAGGCUCUUGGGAC | 43 |
| hsa-miR-126 | UCGUACCGUGAGUAAUAAUGCG | 44 |
| hsa-miR-126* | CAUUAUUACUUUUGGUACGCG | 45 |
| hsa-miR-127-3p | UCGGAUCCGUCUGAGCUUGGCU | 46 |
| hsa-miR-127-5p | CUGAAGCUCAGAGGGCUCUGAU | 47 |
| hsa-miR-128a | UCACAGUGAACCGGUCUCUUU | 48 |
| hsa-miR-128b | UCACAGUGAACCGGUCUCUUU | 49 |
| hsa-miR-129* | AAGCCCUUACCCCAAAAAGUAU | 50 |
| hsa-miR-129-3p | AAGCCCUUACCCCAAAAAGCAU | 51 |
| hsa-miR-129-5p | CUUUUUGCGGUCUGGGCUUGC | 52 |
| hsa-miR-130a | CAGUGCAAUGUUAAAAGGGCAU | 53 |
| hsa-miR-130a* | UUCACAUUGUGCUACUGUCUGC | 54 |
| hsa-miR-130b | CAGUGCAAUGAUGAAAGGGCAU | 55 |
| hsa-miR-130b* | ACUCUUUCCCUGUUGCACUAC | 56 |
| hsa-miR-132 | UAACAGUCUACAGCCAUGGUCG | 57 |
| hsa-miR-132* | ACCGUGGCUUUCGAUUGUUACU | 58 |
| hsa-miR-133a | UUUGGUCCCCUUCAACCAGCUG | 59 |
| hsa-miR-133b | UUUGGUCCCCUUCAACCAGCUA | 60 |
| hsa-miR-134 | UGUGACUGGUUGACCAGAGGGG | 61 |
| hsa-miR-135a | UAUGGCUUUUUAUUCCUAUGUGA | 62 |
| hsa-miR-135a* | UAUAGGGAUUGGAGCCGUGGCG | 63 |
| hsa-miR-135b | UAUGGCUUUUCAUUCCUAUGUGA | 64 |
| hsa-miR-135b* | AUGUAGGGCUAAAAGCCAUGGG | 65 |
| hsa-miR-136 | ACUCCAUUUGUUUUGAUGAUGGA | 66 |
| hsa-miR-136* | CAUCAUCGUCUCAAAUGAGUCU | 67 |
| hsa-miR-137 | UUAUUGCUUAAGAAUACGCGUAG | 68 |
| hsa-miR-138 | AGCUGGUGUUGUGAAUCAGGCCG | 69 |
| hsa-miR-138-1* | GCUACUUCACAACACCAGGGCC | 70 |
| hsa-miR-138-2* | GCUAUUUCACGACACCAGGGUU | 71 |
| hsa-miR-139-3p | GGAGACGCGGCCCUGUUGGAGU | 72 |
| hsa-miR-139-5p | UCUACAGUGCACGUGUCUCCAG | 73 |
| hsa-miR-140-3p | UACCACAGGGUAGAACCACGG | 74 |
| hsa-miR-140-5p | CAGUGGUUUUACCCUAUGGUAG | 75 |
| hsa-miR-141 | UAACACUGUCUGGUAAAGAUGG | 76 |
| hsa-miR-141* | CAUCUUCCAGUACAGUGUUGGA | 77 |
| hsa-miR-142-3p | UGUAGUGUUUCCUACUUUAUGGA | 78 |
| hsa-miR-142-5p | CAUAAAGUAGAAAGCACUACU | 79 |
| hsa-miR-143 | UGAGAUGAAGCACUGUAGCUC | 80 |
| hsa-miR-143* | GGUGCAGUGCUGCAUCUCUGGU | 81 |
| hsa-miR-144 | UACAGUAUAGAUGAUGUACU | 82 |
| hsa-miR-144* | GGAUAUCAUCAUAUACUGUAAG | 83 |
| hsa-miR-145 | GUCCAGUUUUCCCAGGAAUCCCU | 84 |
| hsa-miR-145* | GGAUUCCUGGAAAUACUGUUCU | 85 |
| hsa-miR-146a | UGAGAACUGAAUUCCAUGGGUU | 86 |
| hsa-miR-146a* | CCUCUGAAAUUCAGUUCUUCAG | 87 |
| hsa-miR-146b-3p | UGCCCUGUGGACUCAGUUCUGG | 88 |
| hsa-miR-146b-5p | UGAGAACUGAAUUCCAUAGGCU | 89 |
| hsa-miR-147 | GUGUGUGGAAAUGCUUCUGC | 90 |
| hsa-miR-147b | GUGUGCGGAAAUGCUUCUGCUA | 91 |
| hsa-miR-148a | UCAGUGCACUACAGAACUUUGU | 92 |
| hsa-miR-148a* | AAAGUUCUGAGACACUCCGACU | 93 |
| hsa-miR-148b | UCAGUGCAUCACAGAACUUUGU | 94 |
| hsa-miR-148b* | AAGUUCUGUUAUACACUCAGGC | 95 |
| hsa-miR-149 | UCUGGCUCCGUGUCUUCACUCCC | 96 |
| hsa-miR-149* | AGGGAGGGACGGGGGCUGUGC | 97 |
| hsa-miR-150 | UCUCCCAACCCUUGUACCAGUG | 98 |
| hsa-miR-150* | CUGGUACAGGCCUGGGGGACAG | 99 |
| hsa-miR-151-3p | CUAGACUGAAGCUCCUUGAGG | 100 |
| hsa-miR-151-5p | UCGAGGAGCUCACAGUCUAGU | 101 |
| hsa-miR-152 | UCAGUGCAUGACAGAACUUGG | 102 |
| hsa-miR-153 | UUGCAUAGUCACAAAAGUGAUC | 103 |
| hsa-miR-154 | UAGGUUAUCCGUGUUGCCUUCG | 104 |
| hsa-miR-154* | AAUCAUACACGGUUGACCUAUU | 105 |
| hsa-miR-155 | UUAAUGCUAAUCGUGAUAGGGGU | 106 |
| hsa-miR-155* | CUCCUACAUAUUAGCAUUAACA | 107 |
| hsa-miR-15a | UAGCAGCACAUAAUGGUUUGUG | 108 |
| hsa-miR-15a* | CAGGCCAUAUUGUGCUGCCUCA | 109 |
| hsa-miR-15b | UAGCAGCACAUCAUGGUUUACA | 110 |
| hsa-miR-15b* | CGAAUCAUUAUUUGCUGCUCUA | 111 |
| hsa-miR-16 | UAGCAGCACGUAAAUAUUGGCG | 112 |
| hsa-miR-16-1* | CCAGUAUUAACUGUGCUGCUGA | 113 |
| hsa-miR-16-2* | CCAAUAUUACUGUGCUGCUUUA | 114 |
| hsa-miR-17 | CAAAGUGCUUACAGUGCAGGUAG | 115 |
| hsa-miR-17* | ACUGCAGUGAAGGCACUUGUAG | 116 |
| hsa-miR-181a | AACAUUCAACGCUGUCGGUGAGU | 117 |
| hsa-miR-181a* | ACCAUCGACCGUUGAUUGUACC | 118 |
| hsa-miR-181a-2* | ACCACUGACCGUUGACUGUACC | 119 |
| hsa-miR-181b | AACAUUCAUUGCUGUCGGUGGGU | 120 |
| hsa-miR-181c | AACAUUCAACCUGUCGGUGAGU | 121 |
| hsa-miR-181c* | AACCAUCGACCGUUGAGUGGAC | 122 |
| hsa-miR-181d | AACAUUCAUUGUUGUCGGUGGGU | 123 |
| hsa-miR-182 | UUUGGCAAUGGUAGAACUCACACU | 124 |
| hsa-miR-182* | UGGUUCUAGACUUGCCAACUA | 125 |
| hsa-miR-183 | UAUGGCACUGGUAGAAUUCACU | 126 |
| hsa-miR-183* | GUGAAUUACCGAAGGGCCAUAA | 127 |
| hsa-miR-184 | UGGACGGAGAACUGAUAAGGGU | 128 |
| hsa-miR-185 | UGGAGAGAAAGGCAGUUCCUGA | 129 |
| hsa-miR-185* | AGGGGCUGGCUUUCCUCUGGUC | 130 |
| hsa-miR-186 | CAAAGAAUUCUCCUUUUGGGCU | 131 |
| hsa-miR-186* | GCCCAAAGGUGAAUUUUUUGGG | 132 |
| hsa-miR-187 | UCGUGUCUUGUGUUGCAGCCGG | 133 |
| hsa-miR-187* | GGCUACAACACAGGACCCGGGC | 134 |
| hsa-miR-188-3p | CUCCCACAUGCAGGGUUUGCA | 135 |
| hsa-miR-188-5p | CAUCCCUUGCAUGGUGGAGGG | 136 |
| hsa-miR-18a | UAAGGUGCAUCUAGUGCAGAUAG | 137 |
| hsa-miR-18a* | ACUGCCCUAAGUGCUCCUUCUGG | 138 |
| hsa-miR-18b | UAAGGUGCAUCUAGUGCAGUUAG | 139 |
| hsa-miR-18b* | UGCCCUAAAUGCCCCUUCUGGC | 140 |
| hsa-miR-190 | UGAUAUGUUUGAUAUAUUAGGU | 141 |
| hsa-miR-190b | UGAUAUGUUUGAUAUUGGGUU | 142 |
| hsa-miR-191 | CAACGGAAUCCCAAAAGCAGCUG | 143 |
| hsa-miR-191* | GCUGCGCUUGGAUUUCGUCCCC | 144 |
| hsa-miR-192 | CUGACCUAUGAAUUGACAGCC | 145 |
| hsa-miR-192* | CUGCCAAUUCCAUAGGUCACAG | 146 |
| hsa-miR-193a-3p | AACUGGCCUACAAAGUCCCAGU | 147 |
| hsa-miR-193a-5p | UGGGUCUUUGCGGGCGAGAUGA | 148 |
| hsa-miR-193b | AACUGGCCCUCAAAGUCCCGCU | 149 |
| hsa-miR-193b* | CGGGGUUUUGAGGGCGAGAUGA | 150 |
| hsa-miR-194 | UGUAACAGCAACUCCAUGUGGA | 151 |
| hsa-miR-194* | CCAGUGGGGCUGCUGUUAUCUG | 152 |
| hsa-miR-195 | UAGCAGCACAGAAAUAUUGGC | 153 |
| hsa-miR-195* | CCAAUAUUGGCUGUGCUGCUCC | 154 |
| hsa-miR-196a | UAGGUAGUUUCAUGUUGUUGGG | 155 |
| hsa-miR-196a* | CGGCAACAAGAAACUGCCUGAG | 156 |
| hsa-miR-196b | UAGGUAGUUUCCUGUUGUUGGG | 157 |
| hsa-miR-197 | UUCACCACCUUCUCCACCCAGC | 158 |
| hsa-miR-198 | GGUCCAGAGGGGAGAUAGGUUC | 159 |
| hsa-miR-199a-3p | ACAGUAGUCUGCACAUUGGUUA | 160 |
| hsa-miR-199a-5p | CCCAGUGUUCAGACUACCUGUUC | 161 |
| hsa-miR-199b-3p | ACAGUAGUCUGCACAUUGGUUA | 162 |
| hsa-miR-199b-5p | CCCAGUGUUUAGACUAUCUGUUC | 163 |
| hsa-miR-19a | UGUGCAAAUCUAUGCAAAACUGA | 164 |
| hsa-miR-19a* | AGUUUUGCAUAGUUGCACUACA | 165 |
| hsa-miR-19b | UGUGCAAAUCCAUGCAAAACUGA | 166 |
| hsa-miR-19b-1* | AGUUUUGCAGGUUUGCAUCCAGC | 167 |
| hsa-miR-19b-2* | AGUUUUGCAGGUUUGCAUUUCA | 168 |
| hsa-miR-200a | UAACACUGUCUGGUAACGAUGU | 169 |
| hsa-miR-200a* | CAUCUUACCGGACAGUGCUGGA | 170 |
| hsa-miR-200b | UAAUACUGCCUGGUAAUGAUGA | 171 |
| hsa-miR-200b* | CAUCUUACUGGGCAGCAUUGGA | 172 |
| hsa-miR-200c | UAAUACUGCCGGGUAAUGAUGGA | 173 |
| hsa-miR-200c* | CGUCUUACCCAGCAGUGUUUGG | 174 |
| hsa-miR-202 | AGAGGUAUAGGGCAUGGGAA | 175 |
| hsa-miR-202* | UUCCUAUGCAUAUACUUCUUUG | 176 |
| hsa-miR-203 | GUGAAAUGUUUAGGACCACUAG | 177 |
| hsa-miR-204 | UUCCCUUUGUCAUCCUAUGCCU | 178 |
| hsa-miR-205 | UCCUUCAUUCCACCGGAGUCUG | 179 |
| hsa-miR-206 | UGGAAUGUAAGGAAGUGUGUGG | 180 |
| hsa-miR-208 | AUAAGACGAGCAAAAAGCUUGU | 181 |
| hsa-miR-208b | AUAAGACGAACAAAAGGUUUGU | 182 |
| hsa-miR-20a | UAAAGUGCUUAUAGUGCAGGUAG | 183 |
| hsa-miR-20a* | ACUGCAUUAUGAGCACUUAAAG | 184 |
| hsa-miR-20b | CAAAGUGCUCAUAGUGCAGGUAG | 185 |
| hsa-miR-20b* | ACUGUAGUAUGGGCACUUCCAG | 186 |
| hsa-miR-21 | UAGCUUAUCAGACUGAUGUUGA | 187 |
| hsa-miR-21* | CAACACCAGUCGAUGGGCUGU | 188 |
| hsa-miR-210 | CUGUGCGUGUGACAGCGGCUGA | 189 |
| hsa-miR-211 | UUCCCUUUGUCAUCCUUCGCCU | 190 |
| hsa-miR-212 | UAACAGUCUCCAGUCACGGCC | 191 |
| hsa-miR-214 | ACAGCAGGCACAGACAGGCAGU | 192 |
| hsa-miR-214* | UGCCUGUCUACACUUGCUGUGC | 193 |
| hsa-miR-215 | AUGACCUAUGAAUUGACAGAC | 194 |
| hsa-miR-216a | UAAUCUCAGCUGGCAACUGUGA | 195 |
| hsa-miR-216b | AAAUCUCUGCAGGCAAAUGUGA | 196 |
| hsa-miR-217 | UACUGCAUCAGGAACUGAUUGGA | 197 |
| hsa-miR-218 | UUGUGCUUGAUCUAACCAUGU | 198 |
| hsa-miR-218-1* | AUGGUUCCGUCAAGCACCAUGG | 199 |
| hsa-miR-218-2* | CAUGGUUCUGUCAAGCACCGCG | 200 |
| hsa-miR-219-1-3p | AGAGUUGAGUCUGGACGUCCCG | 201 |
| hsa-miR-219-2-3p | AGAAUUGUGGCUGGACAUCUGU | 202 |
| hsa-miR-219-5p | UGAUUGUCCAAACGCAAUUCU | 203 |
| hsa-miR-22 | AAGCUGCCAGUUGAAGAACUGU | 204 |
| hsa-miR-22* | AGUUCUUCAGUGGCAAGCUUUA | 205 |
| hsa-miR-220 | CCACACCGUAUCUGACACUUU | 206 |
| hsa-miR-220b | CCACCACCGUGUCUGACACUU | 207 |
| hsa-miR-220c | ACACAGGGCUGUUGUGAAGACU | 208 |
| hsa-miR-221 | AGCUACAUUGUCUGCUGGGUUUC | 209 |
| hsa-miR-221* | ACCUGGCAUACAAUGUAGAUUU | 210 |
| hsa-miR-222 | AGCUACAUCUGGCUACUGGGU | 211 |
| hsa-miR-222* | CUCAGUAGCCAGUGUAGAUCCU | 212 |
| hsa-miR-223 | UGUCAGUUUGUCAAAUACCCCA | 213 |
| hsa-miR-223* | CGUGUAUUUGACAAGCUGAGUU | 214 |
| hsa-miR-224 | CAAGUCACUAGUGGUUCCGUU | 215 |
| hsa-miR-23a | AUCACAUUGCCAGGGAUUUCC | 216 |
| hsa-miR-23a* | GGGGUUCCUGGGGAUGGGAUUU | 217 |
| hsa-miR-23b | AUCACAUUGCCAGGGAUUACC | 218 |
| hsa-miR-23b* | UGGGUUCCUGGCAUGCUGAUUU | 219 |
| hsa-miR-24 | UGGCUCAGUUCAGCAGGAACAG | 220 |
| hsa-miR-24-1* | UGCCUACUGAGCUGAUAUCAGU | 221 |
| hsa-miR-24-2* | UGCCUACUGAGCUGAAACACAG | 222 |
| hsa-miR-25 | CAUUGCACUUGUCUCGGUCUGA | 223 |
| hsa-miR-25* | AGGCGGAGACUUGGGCAAUUG | 224 |
| hsa-miR-26a | UUCAAGUAAUCCAGGAUAGGCU | 225 |
| hsa-miR-26a-1* | CCUAUUCUUGGUUACUUGCACG | 226 |
| hsa-miR-26a-2* | CCUAUUCUUGAUUACUUGUUUC | 227 |
| hsa-miR-26b | UUCAAGUAAUUCAGGAUAGGU | 228 |
| hsa-miR-26b* | CCUGUUCUCCAUUACUUGGCUC | 229 |
| hsa-miR-27a | UUCACAGUGGCUAAGUUCCGC | 230 |
| hsa-miR-27a* | AGGGCUUAGCUGCUUGUGAGCA | 231 |
| hsa-miR-27b | UUCACAGUGGCUAAGUUCUGC | 232 |
| hsa-miR-27b* | AGAGCUUAGCUGAUUGGUGAAC | 233 |
| hsa-miR-28-3p | CACUAGAUUGUGAGCUCCUGGA | 234 |
| hsa-miR-28-5p | AAGGAGCUCACAGUCUAUUGAG | 235 |
| hsa-miR-296-3p | GAGGGUUGGGUGGAGGCUCUCC | 236 |
| hsa-miR-296-5p | AGGGCCCCCCCUCAAUCCUGU | 237 |
| hsa-miR-297 | AUGUAUGUGUGCAUGUGCAUG | 238 |
| hsa-miR-298 | AGCAGAAGCAGGGAGGUUCUCCCA | 239 |
| hsa-miR-299-3p | UAUGUGGGAUGGUAAACCGCUU | 240 |
| hsa-miR-299-5p | UGGUUUACCGUCCCACAUACAU | 241 |
| hsa-miR-29a | UAGCACCAUCUGAAAUCGGUUA | 242 |
| hsa-miR-29a* | ACUGAUUUCUUUUGGUGUUCAG | 243 |
| hsa-miR-29b | UAGCACCAUUUGAAAUCAGUGUU | 244 |
| hsa-miR-29b-1* | GCUGGUUUCAUAUGGUGGUUUAGA | 245 |
| hsa-miR-29b-2* | CUGGUUUCACAUGGUGGCUUAG | 246 |
| hsa-miR-29c | UAGCACCAUUUGAAAUCGGUUA | 247 |
| hsa-miR-29c* | UGACCGAUUUCUCCUGGUGUUC | 248 |
| hsa-miR-300 | UAUACAAGGGCAGACUCUCUCU | 249 |
| hsa-miR-301a | CAGUGCAAUAGUAUUGUCAAAGC | 250 |
| hsa-miR-301b | CAGUGCAAUGAUAUUGUCAAAGC | 251 |
| hsa-miR-302a | UAAGUGCUUCCAUGUUUUGGUGA | 252 |
| hsa-miR-302a* | ACUUAAACGUGGAUGUACUUGCU | 253 |
| hsa-miR-302b | UAAGUGCUUCCAUGUUUUAGUAG | 254 |
| hsa-miR-302b* | ACUUUAACAUGGAAGUGCUUUC | 255 |
| hsa-miR-302c | UAAGUGCUUCCAUGUUUCAGUGG | 256 |
| hsa-miR-302c* | UUUAACAUGGGGGUACCUGCUG | 257 |
| hsa-miR-302d | UAAGUGCUUCCAUGUUUGAGUGU | 258 |
| hsa-miR-302d* | ACUUUAACAUGGAGGCACUUGC | 259 |
| hsa-miR-30a | UGUAAACAUCCUCGACUGGAAG | 260 |
| hsa-miR-30a* | CUUUCAGUCGGAUGUUUGCAGC | 261 |
| hsa-miR-30b | UGUAAACAUCCUACACUCAGCU | 262 |
| hsa-miR-30b* | CUGGGAGGUGGAUGUUUACUUC | 263 |
| hsa-miR-30c | UGUAAACAUCCUACACUCUCAGC | 264 |
| hsa-miR-30c-1* | CUGGGAGAGGGUUGUUUACUCC | 265 |
| hsa-miR-30c-2* | CUGGGAGAAGGCUGUUUACUCU | 266 |
| hsa-miR-30d | UGUAAACAUCCCCGACUGGAAG | 267 |
| hsa-miR-30d* | CUUUCAGUCAGAUGUUUGCUGC | 268 |
| hsa-miR-30e | UGUAAACAUCCUUGACUGGAAG | 269 |
| hsa-miR-30e* | CUUUCAGUCGGAUGUUUACAGC | 270 |
| hsa-miR-31 | AGGCAAGAUGCUGGCAUAGCU | 271 |
| hsa-miR-31* | UGCUAUGCCAACAUAUUGCCAU | 272 |
| hsa-miR-32 | UAUUGCACAUUACUAAGUUGCA | 273 |
| hsa-miR-32* | CAAUUUAGUGUGUGUGAUAUUU | 274 |
| hsa-miR-320 | AAAAGCUGGGUUGAGAGGGCGA | 275 |
| hsa-miR-323-3p | CACAUUACACGGUCGACCUCU | 276 |
| hsa-miR-323-5p | AGGUGGUCCGUGGCGCGUUCGC | 277 |
| hsa-miR-324-3p | ACUGCCCCAGGUGCUGCUGG | 278 |
| hsa-miR-324-5p | CGCAUCCCCUAGGGCAUUGGUGU | 279 |
| hsa-miR-325 | CCUAGUAGGUGUCCAGUAAGUGU | 280 |
| hsa-miR-326 | CCUCUGGGCCCUUCCUCCAG | 281 |
| hsa-miR-328 | CUGGCCCUCUCUGCCCUUCCGU | 282 |
| hsa-miR-329 | AACACACCUGGUUAACCUCUUU | 283 |
| hsa-miR-330-3p | GCAAAGCACACGGCCUGCAGAGA | 284 |
| hsa-miR-330-5p | UCUCUGGGCCUGUGUCUUAGGC | 285 |
| hsa-miR-331-3p | GCCCCUGGGCCUAUCCUAGAA | 286 |
| hsa-miR-331-5p | CUAGGUAUGGUCCCAGGGAUCC | 287 |
| hsa-miR-335 | UCAAGAGCAAUAACGAAAAAUGU | 288 |
| hsa-miR-335* | UUUUUCAUUAUUGCUCCUGACC | 289 |
| hsa-miR-337-3p | CUCCUAUAUGAUGCCUUUCUUC | 290 |
| hsa-miR-337-5p | GAACGGCUUCAUACAGGAGUU | 291 |
| hsa-miR-338-3p | UCCAGCAUCAGUGAUUUUGUUG | 292 |
| hsa-miR-338-5p | AACAAUAUCCUGGUGCUGAGUG | 293 |
| hsa-miR-339-3p | UGAGCGCCUCGACGACAGAGCCG | 294 |
| hsa-miR-339-5p | UCCCUGUCCUCCAGGAGCUCACG | 295 |
| hsa-miR-33a | GUGCAUUGUAGUUGCAUUGCA | 296 |
| hsa-miR-33a* | CAAUGUUUCCACAGUGCAUCAC | 297 |
| hsa-miR-33b | GUGCAUUGCUGUUGCAUUGC | 298 |
| hsa-miR-33b* | CAGUGCCUCGGCAGUGCAGCCC | 299 |
| hsa-miR-340 | UUAUAAAGCAAUGAGACUGAUU | 300 |
| hsa-miR-340* | UCCGUCUCAGUUACUUUAUAGC | 301 |
| hsa-miR-342-3p | UCUCACACAGAAAUCGCACCCGU | 302 |
| hsa-miR-342-5p | AGGGGUGCUAUCUGUGAUUGA | 303 |
| hsa-miR-345 | GCUGACUCCUAGUCCAGGGCUC | 304 |
| hsa-miR-346 | UGUCUGCCCGCAUGCCUGCCUCU | 305 |
| hsa-miR-34a | UGGCAGUGUCUUAGCUGGUUGU | 306 |
| hsa-miR-34a* | CAAUCAGCAAGUAUACUGCCCU | 307 |
| hsa-miR-34b | CAAUCACUAACUCCACUGCCAU | 308 |
| hsa-miR-34b* | UAGGCAGUGUCAUUAGCUGAUUG | 309 |
| hsa-miR-34c-3p | AAUCACUAACCACACGGCCAGG | 310 |
| hsa-miR-34c-5p | AGGCAGUGUAGUUAGCUGAUUGC | 311 |
| hsa-miR-361-3p | UCCCCCAGGUGUGAUUCUGAUUU | 312 |
| hsa-miR-361-5p | UUAUCAGAAUCUCCAGGGGUAC | 313 |
| hsa-miR-362-3p | AACACACCUAUUCAAGGAUUCA | 314 |
| hsa-miR-362-5p | AAUCCUUGGAACCUAGGUGUGAGU | 315 |
| hsa-miR-363 | AAUUGCACGGUAUCCAUCUGUA | 316 |
| hsa-miR-363* | CGGGUGGAUCACGAUGCAAUUU | 317 |
| hsa-miR-365 | UAAUGCCCCUAAAAAUCCUUAU | 318 |
| hsa-miR-367 | AAUUGCACUUUAGCAAUGGUGA | 319 |
| hsa-miR-367* | ACUGUUGCUAAUAUGCAACUCU | 320 |
| hsa-miR-369-3p | AAUAAUACAUGGUUGAUCUUU | 321 |
| hsa-miR-369-5p | AGAUCGACCGUGUUAUAUUCGC | 322 |
| hsa-miR-370 | GCCUGCUGGGGUGGAACCUGGU | 323 |
| hsa-miR-371-3p | AAGUGCCGCCAUCUUUUGAGUGU | 324 |
| hsa-miR-371-5p | ACUCAAACUGUGGGGGCACU | 325 |
| hsa-miR-372 | AAAGUGCUGCGACAUUUGAGCGU | 326 |
| hsa-miR-373 | GAAGUGCUUCGAUUUUGGGGUGU | 327 |
| hsa-miR-373* | ACUCAAAAUGGGGGCGCUUUCC | 328 |
| hsa-miR-374a | UUAUAAUACAACCUGAUAAGUG | 329 |
| hsa-miR-374a* | CUUAUCAGAUUGUAUUGUAAUU | 330 |
| hsa-miR-374b | AUAUAAUACAACCUGCUAAGUG | 331 |
| hsa-miR-374b* | CUUAGCAGGUUGUAUUAUCAUU | 332 |
| hsa-miR-375 | UUUGUUCGUUCGGCUCGCGUGA | 333 |
| hsa-miR-376a | AUCAUAGAGGAAAAUCCACGU | 334 |
| hsa-miR-376a* | GUAGAUUCUCCUUCUAUGAGUA | 335 |
| hsa-miR-376b | AUCAUAGAGGAAAAUCCAUGUU | 336 |
| hsa-miR-376c | AACAUAGAGGAAAUUCCACGU | 337 |
| hsa-miR-377 | AUCACACAAAGGCAACUUUUGU | 338 |
| hsa-miR-377* | AGAGGUUGCCCUUGGUGAAUUC | 339 |
| hsa-miR-378 | ACUGGACUUGGAGUCAGAAGG | 340 |
| hsa-miR-378* | CUCCUGACUCCAGGUCCUGUGU | 341 |
| hsa-miR-379 | UGGUAGACUAUGGAACGUAGG | 342 |
| hsa-miR-379* | UAUGUAACAUGGUCCACUAACU | 343 |
| hsa-miR-380 | UAUGUAAUAUGGUCCACAUCUU | 344 |
| hsa-miR-380* | UGGUUGACCAUAGAACAUGCGC | 345 |
| hsa-miR-381 | UAUACAAGGGCAAGCUCUCUGU | 346 |
| hsa-miR-382 | GAAGUUGUUCGUGGUGGAUUCG | 347 |
| hsa-miR-383 | AGAUCAGAAGGUGAUUGUGGCU | 348 |
| hsa-miR-384 | AUUCCUAGAAAUUGUUCAUA | 349 |
| hsa-miR-409-3p | GAAUGUUGCUCGGUGAACCCCU | 350 |
| hsa-miR-409-5p | AGGUUACCCGAGCAACUUUGCAU | 351 |
| hsa-miR-410 | AAUAUAACACAGAUGGCCUGU | 352 |
| hsa-miR-411 | UAGUAGACCGUAUAGCGUACG | 353 |
| hsa-miR-411* | UAUGUAACACGGUCCACUAACC | 354 |
| hsa-miR-412 | ACUUCACCUGGUCCACUAGCCGU | 355 |
| hsa-miR-421 | AUCAACAGACAUUAAUUGGGCGC | 356 |
| hsa-miR-422a | ACUGGACUUAGGGUCAGAAGGC | 357 |
| hsa-miR-423-3p | AGCUCGGUCUGAGGCCCCUCAGU | 358 |
| hsa-miR-423-5p | UGAGGGGCAGAGAGCGAGACUUU | 359 |
| hsa-miR-424 | CAGCAGCAAUUCAUGUUUUGAA | 360 |
| hsa-miR-424* | CAAAACGUGAGGCGCUGCUAU | 361 |
| hsa-miR-425 | AAUGACACGAUCACUCCCGUUGA | 362 |
| hsa-miR-425* | AUCGGGAAUGUCGUGUCCGCCC | 363 |
| hsa-miR-429 | UAAUACUGUCUGGUAAAACCGU | 364 |
| hsa-miR-431 | UGUCUUGCAGGCCGUCAUGCA | 365 |
| hsa-miR-431* | CAGGUCGUCUUGCAGGGCUUCU | 366 |
| hsa-miR-432 | UCUUGGAGUAGGUCAUUGGGUGG | 367 |
| hsa-miR-432* | CUGGAUGGCUCCUCCAUGUCU | 368 |
| hsa-miR-433 | AUCAUGAUGGGCUCCUCGGUGU | 369 |
| hsa-miR-448 | UUGCAUAUGUAGGAUGUCCCAU | 370 |
| hsa-miR-449a | UGGCAGUGUAUUGUUAGCUGGU | 371 |
| hsa-miR-449b | AGGCAGUGUAUUGUUAGCUGGC | 372 |
| hsa-miR-450a | UUUUGCGAUGUGUUCCUAAUAU | 373 |
| hsa-miR-450b-3p | UUGGGAUCAUUUUGCAUCCAUA | 374 |
| hsa-miR-450b-5p | UUUUGCAAUAUGUUCCUGAAUA | 375 |
| hsa-miR-451 | AAACCGUUACCAUUACUGAGUU | 376 |
| hsa-miR-452 | AACUGUUUGCAGAGGAAACUGA | 377 |
| hsa-miR-452* | CUCAUCUGCAAAGAAGUAAGUG | 378 |
| hsa-miR-453 | AGGUUGUCCGUGGUGAGUUCGCA | 379 |
| hsa-miR-454 | UAGUGCAAUAUUGCUUAUAGGGU | 380 |
| hsa-miR-454* | ACCCUAUCAAUAUUGUCUCUGC | 381 |
| hsa-miR-455-3p | GCAGUCCAUGGGCAUAUACAC | 382 |
| hsa-miR-455-5p | UAUGUGCCUUUGGACUACAUCG | 383 |
| hsa-miR-483-3p | UCACUCCUCUCCUCCCGUCUU | 384 |
| hsa-miR-483-5p | AAGACGGGAGGAAAGAAGGGAG | 385 |
| hsa-miR-484 | UCAGGCUCAGUCCCCUCCCGAU | 386 |
| hsa-miR-485-3p | GUCAUACACGGCUCUCCUCUCU | 387 |
| hsa-miR-485-5p | AGAGGCUGGCCGUGAUGAAUUC | 388 |
| hsa-miR-486-3p | CGGGGCAGCUCAGUACAGGAU | 389 |
| hsa-miR-486-5p | UCCUGUACUGAGCUGCCCCGAG | 390 |
| hsa-miR-487a | AAUCAUACAGGGACAUCCAGUU | 391 |
| hsa-miR-487b | AAUCGUACAGGGUCAUCCACUU | 392 |
| hsa-miR-488 | UUGAAAGGCUAUUUCUUGGUC | 393 |
| hsa-miR-488* | CCCAGAUAAUGGCACUCUCAA | 394 |
| hsa-miR-489 | GUGACAUCACAUAUACGGCAGC | 395 |
| hsa-miR-490-3p | CAACCUGGAGGACUCCAUGCUG | 396 |
| hsa-miR-490-5p | CCAUGGAUCUCCAGGUGGGU | 397 |
| hsa-miR-491-3p | CUUAUGCAAGAUUCCCUUCUAC | 398 |
| hsa-miR-491-5p | AGUGGGGAACCCUUCCAUGAGG | 399 |
| hsa-miR-492 | AGGACCUGCGGGACAAGAUUCUU | 400 |
| hsa-miR-493 | UGAAGGUCUACUGUGUGCCAGG | 401 |
| hsa-miR-493* | UUGUACAUGGUAGGCUUUCAUU | 402 |
| hsa-miR-494 | UGAAACAUACACGGGAAACCUC | 403 |
| hsa-miR-495 | AAACAAACAUGGUGCACUUCUU | 404 |
| hsa-miR-496 | UGAGUAUUACAUGGCCAAUCUC | 405 |
| hsa-miR-497 | CAGCAGCACACUGUGGUUUGU | 406 |
| hsa-miR-497* | CAAACCACACUGUGGUGUUAGA | 407 |
| hsa-miR-498 | UUUCAAGCCAGGGGGCGUUUUUC | 408 |
| hsa-miR-499-3p | AACAUCACAGCAAGUCUGUGCU | 409 |
| hsa-miR-499-5p | UUAAGACUUGCAGUGAUGUUU | 410 |
| hsa-miR-500 | UAAUCCUUGCUACCUGGGUGAGA | 411 |
| hsa-miR-500* | AUGCACCUGGGCAAGGAUUCUG | 412 |
| hsa-miR-501-3p | AAUGCACCCGGGCAAGGAUUCU | 413 |
| hsa-miR-501-5p | AAUCCUUUGUCCCUGGGUGAGA | 414 |
| hsa-miR-502-3p | AAUGCACCUGGGCAAGGAUUCA | 415 |
| hsa-miR-502-5p | AUCCUUGCUAUCUGGGUGCUA | 416 |
| hsa-miR-503 | UAGCAGCGGGAACAGUUCUGCAG | 417 |
| hsa-miR-504 | AGACCCUGGUCUGCACUCUAUC | 418 |
| hsa-miR-505 | CGUCAACACUUGCUGGUUUCCU | 419 |
| hsa-miR-505* | GGGAGCCAGGAAGUAUUGAUGU | 420 |
| hsa-miR-506 | UAAGGCACCCUUCUGAGUAGA | 421 |
| hsa-miR-507 | UUUUGCACCUUUUGGAGUGAA | 422 |
| hsa-miR-508-3p | UGAUUGUAGCCUUUUGGAGUAGA | 423 |
| hsa-miR-508-5p | UACUCCAGAGGGCGUCACUCAUG | 424 |
| hsa-miR-509-3-5p | UACUGCAGACGUGGCAAUCAUG | 425 |
| hsa-miR-509-3p | UGAUUGGUACGUCUGUGGGUAG | 426 |
| hsa-miR-509-5p | UACUGCAGACAGUGGCAAUCA | 427 |
| hsa-miR-510 | UACUCAGGAGAGUGGCAAUCAC | 428 |
| hsa-miR-511 | GUGUCUUUUGCUCUGCAGUCA | 429 |
| hsa-miR-512-3p | AAGUGCUGUCAUAGCUGAGGUC | 430 |
| hsa-miR-512-5p | CACUCAGCCUUGAGGGCACUUUC | 431 |
| hsa-miR-513-3p | UAAAUUUCACCUUUCUGAGAAGG | 432 |
| hsa-miR-513-5p | UUCACAGGGAGGUGUCAU | 433 |
| hsa-miR-514 | AUUGACACUUCUGUGAGUAGA | 434 |
| hsa-miR-515-3p | GAGUGCCUUCUUUUGGAGCGUU | 435 |
| hsa-miR-515-5p | UUCUCCAAAAGAAAGCACUUUCUG | 436 |
| hsa-miR-516a-3p | UGCUUCCUUUCAGAGGGU | 437 |
| hsa-miR-516a-5p | UUCUCGAGGAAAGAAGCACUUUC | 438 |
| hsa-miR-516b | AUCUGGAGGUAAGAAGCACUUU | 439 |
| hsa-miR-516b* | UGCUUCCUUUCAGAGGGU | 440 |
| hsa-miR-517* | CCUCUAGAUGGAAGCACUGUCU | 441 |
| hsa-miR-517a | AUCGUGCAUCCCUUUAGAGUGU | 442 |
| hsa-miR-517b | UCGUGCAUCCCUUUAGAGUGUU | 443 |
| hsa-miR-517c | AUCGUGCAUCCUUUUAGAGUGU | 444 |
| hsa-miR-518a-3p | GAAAGCGCUUCCCUUUGCUGGA | 445 |
| hsa-miR-518a-5p | CUGCAAAGGGAAGCCCUUUC | 446 |
| hsa-miR-518b | CAAAGCGCUCCCCUUUAGAGGU | 447 |
| hsa-miR-518c | CAAAGCGCUUCUCUUUAGAGUGU | 448 |
| hsa-miR-518c* | UCUCUGGAGGGAAGCACUUUCUG | 449 |
| hsa-miR-518d-3p | CAAAGCGCUUCCCUUUGGAGC | 450 |
| hsa-miR-518d-5p | CUCUAGAGGGAAGCACUUUCUG | 451 |
| hsa-miR-518e | AAAGCGCUUCCCUUCAGAGUG | 452 |
| hsa-miR-518e* | CUCUAGAGGGAAGCGCUUUCUG | 453 |
| hsa-miR-518f | GAAAGCGCUUCUCUUUAGAGG | 454 |
| hsa-miR-518f* | CUCUAGAGGGAAGCACUUUCUC | 455 |
| hsa-miR-519a | AAAGUGCAUCCUUUUAGAGUGU | 456 |
| hsa-miR-519a* | CUCUAGAGGGAAGCGCUUUCUG | 457 |
| hsa-miR-519b-3p | AAAGUGCAUCCUUUUAGAGGUU | 458 |
| hsa-miR-519b-5p | CUCUAGAGGGAAGCGCUUUCUG | 459 |
| hsa-miR-519c-3p | AAAGUGCAUCUUUUUAGAGGAU | 460 |
| hsa-miR-519c-5p | CUCUAGAGGGAAGCGCUUUCUG | 461 |
| hsa-miR-519d | CAAAGUGCCUCCCUUUAGAGUG | 462 |
| hsa-miR-519e | AAGUGCCUCCUUUUAGAGUGUU | 463 |
| hsa-miR-519e* | UUCUCCAAAAGGGAGCACUUUC | 464 |
| hsa-miR-520a-3p | AAAGUGCUUCCCUUUGGACUGU | 465 |
| hsa-miR-520a-5p | CUCCAGAGGGAAGUACUUUCU | 466 |
| hsa-miR-520b | AAAGUGCUUCCUUUUAGAGGG | 467 |
| hsa-miR-520c-3p | AAAGUGCUUCCUUUUAGAGGGU | 468 |
| hsa-miR-520c-5p | CUCUAGAGGGAAGCACUUUCUG | 469 |
| hsa-miR-520d-3p | AAAGUGCUUCUCUUUGGUGGGU | 470 |
| hsa-miR-520d-5p | CUACAAAGGGAAGCCCUUUC | 471 |
| hsa-miR-520e | AAAGUGCUUCCUUUUUGAGGG | 472 |
| hsa-miR-520f | AAGUGCUUCCUUUUAGAGGGUU | 473 |
| hsa-miR-520g | ACAAAGUGCUUCCCUUUAGAGUGU | 474 |
| hsa-miR-520h | ACAAAGUGCUUCCCUUUAGAGU | 475 |
| hsa-miR-521 | AACGCACUUCCCUUUAGAGUGU | 476 |
| hsa-miR-522 | AAAAUGGUUCCCUUUAGAGUGU | 477 |
| hsa-miR-522* | CUCUAGAGGGAAGCGCUUUCUG | 478 |
| hsa-miR-523 | GAACGCGCUUCCCUAUAGAGGGU | 479 |
| hsa-miR-523* | CUCUAGAGGGAAGCGCUUUCUG | 480 |
| hsa-miR-524-3p | GAAGGCGCUUCCCUUUGGAGU | 481 |
| hsa-miR-524-5p | CUACAAAGGGAAGCACUUUCUC | 482 |
| hsa-miR-525-3p | GAAGGCGCUUCCCUUUAGAGCG | 483 |
| hsa-miR-525-5p | CUCCAGAGGGAUGCACUUUCU | 484 |
| hsa-miR-526a | CUCUAGAGGGAAGCACUUUCUG | 485 |
| hsa-miR-526b | CUCUUGAGGGAAGCACUUUCUGU | 486 |
| hsa-miR-526b* | GAAAGUGCUUCCUUUUAGAGGC | 487 |
| hsa-miR-527 | CUGCAAAGGGAAGCCCUUUC | 488 |
| hsa-miR-532-3p | CCUCCCACACCCAAGGCUUGCA | 489 |
| hsa-miR-532-5p | CAUGCCUUGAGUGUAGGACCGU | 490 |
| hsa-miR-539 | GGAGAAAUUAUCCUUGGUGUGU | 491 |
| hsa-miR-541 | UGGUGGGCACAGAAUCUGGACU | 492 |
| hsa-miR-541* | AAAGGAUUCUGCUGUCGGUCCCACU | 493 |
| hsa-miR-542-3p | UGUGACAGAUUGAUAACUGAAA | 494 |
| hsa-miR-542-5p | UCGGGGAUCAUCAUGUCACGAGA | 495 |
| hsa-miR-543 | AAACAUUCGCGGUGCACUUCUU | 496 |
| hsa-miR-544 | AUUCUGCAUUUUUAGCAAGUUC | 497 |
| hsa-miR-545 | UCAGCAAACAUUUAUUGUGUGC | 498 |
| hsa-miR-545* | UCAGUAAAUGUUUAUUAGAUGA | 499 |
| hsa-miR-548a-3p | CAAAACUGGCAAUUACUUUUGC | 500 |
| hsa-miR-548a-5p | AAAAGUAAUUGCGAGUUUUACC | 501 |
| hsa-miR-548b-3p | CAAGAACCUCAGUUGCUUUUGU | 502 |
| hsa-miR-548b-5p | AAAAGUAAUUGUGGUUUUGGCC | 503 |
| hsa-miR-548c-3p | CAAAAAUCUCAAUUACUUUUGC | 504 |
| hsa-miR-548c-5p | AAAAGUAAUUGCGGUUUUUGCC | 505 |
| hsa-miR-548d-3p | CAAAAACCACAGUUUCUUUUGC | 506 |
| hsa-miR-548d-5p | AAAAGUAAUUGUGGUUUUUGCC | 507 |
| hsa-miR-549 | UGACAACUAUGGAUGAGCUCU | 508 |
| hsa-miR-550 | AGUGCCUGAGGGAGUAAGAGCCC | 509 |
| hsa-miR-550* | UGUCUUACUCCCUCAGGCACAU | 510 |
| hsa-miR-551a | GCGACCCACUCUUGGUUUCCA | 511 |
| hsa-miR-551b | GCGACCCAUACUUGGUUUCAG | 512 |
| hsa-miR-551b* | GAAAUCAAGCGUGGGUGAGACC | 513 |
| hsa-miR-552 | AACAGGUGACUGGUUAGACAA | 514 |
| hsa-miR-553 | AAAACGGUGAGAUUUUGUUUU | 515 |
| hsa-miR-554 | GCUAGUCCUGACUCAGCCAGU | 516 |
| hsa-miR-555 | AGGGUAAGCUGAACCUCUGAU | 517 |
| hsa-miR-556-3p | AUAUUACCAUUAGCUCAUCUUU | 518 |
| hsa-miR-556-5p | GAUGAGCUCAUUGUAAUAUGAG | 519 |
| hsa-miR-557 | GUUUGCACGGGUGGGCCUUGUCU | 520 |
| hsa-miR-558 | UGAGCUGCUGUACCAAAAU | 521 |
| hsa-miR-559 | UAAAGUAAAUAUGCACCAAAA | 522 |
| hsa-miR-560 | GCGUGCGCCGGCCGGCCGCC | 523 |
| hsa-miR-561 | CAAAGUUUAAGAUCCUUGAAGU | 524 |
| hsa-miR-562 | AAAGUAGCUGUACCAUUUGC | 525 |
| hsa-miR-563 | AGGUUGACAUACGUUUCCC | 526 |
| hsa-miR-564 | AGGCACGGUGUCAGCAGGC | 527 |
| hsa-miR-565 | GGCUGGCUCGCGAUGUCUGUUU | 528 |
| hsa-miR-566 | GGGCGCCUGUGAUCCCAAC | 529 |
| hsa-miR-567 | AGUAUGUUCUUCCAGGACAGAAC | 530 |
| hsa-miR-568 | AUGUAUAAAUGUAUACACAC | 531 |
| hsa-miR-569 | AGUUAAUGAAUCCUGGAAAGU | 532 |
| hsa-miR-570 | CGAAAACAGCAAUUACCUUUGC | 533 |
| hsa-miR-571 | UGAGUUGGCCAUCUGAGUGAG | 534 |
| hsa-miR-572 | GUCCGCUCGGCGGUGGCCCA | 535 |
| hsa-miR-573 | CUGAAGUGAUGUGUAACUGAUCAG | 536 |
| hsa-miR-574-3p | CACGCUCAUGCACACACCCACA | 537 |
| hsa-miR-574-5p | UGAGUGUGUGUGUGUGAGUGUGU | 538 |
| hsa-miR-575 | GAGCCAGUUGGACAGGAGC | 539 |
| hsa-miR-576-3p | AAGAUGUGGAAAAAUUGGAAUC | 540 |
| hsa-miR-576-5p | AUUCUAAUUUCUCCACGUCUUU | 541 |
| hsa-miR-577 | UAGAUAAAAUAUUGGUACCUG | 542 |
| hsa-miR-578 | CUUCUUGUGCUCUAGGAUUGU | 543 |
| hsa-miR-579 | UUCAUUUGGUAUAAACCGCGAUU | 544 |
| hsa-miR-580 | UUGAGAAUGAUGAAUCAUUAGG | 545 |
| hsa-miR-581 | UCUUGUGUUCUCUAGAUCAGU | 546 |
| hsa-miR-582-3p | UAACUGGUUGAACAACUGAACC | 547 |
| hsa-miR-582-5p | UUACAGUUGUUCAACCAGUUACU | 548 |
| hsa-miR-583 | CAAAGAGGAAGGUCCCAUUAC | 549 |
| hsa-miR-584 | UUAUGGUUUGCCUGGGACUGAG | 550 |
| hsa-miR-585 | UGGGCGUAUCUGUAUGCUA | 551 |
| hsa-miR-586 | UAUGCAUUGUAUUUUUAGGUCC | 552 |
| hsa-miR-587 | UUUCCAUAGGUGAUGAGUCAC | 553 |
| hsa-miR-588 | UUGGCCACAAUGGGUUAGAAC | 554 |
| hsa-miR-589 | UGAGAACCACGUCUGCUCUGAG | 555 |
| hsa-miR-589* | UCAGAACAAAUGCCGGUUCCCAGA | 556 |
| hsa-miR-590-3p | UAAUUUUAUGUAUAAGCUAGU | 557 |
| hsa-miR-590-5p | GAGCUUAUUCAUAAAAGUGCAG | 558 |
| hsa-miR-591 | AGACCAUGGGUUCUCAUUGU | 559 |
| hsa-miR-592 | UUGUGUCAAUAUGCGAUGAUGU | 560 |
| hsa-miR-593 | UGUCUCUGCUGGGGUUUCU | 561 |
| hsa-miR-593* | AGGCACCAGCCAGGCAUUGCUCAGC | 562 |
| hsa-miR-595 | GAAGUGUGCCGUGGUGUGUCU | 563 |
| hsa-miR-596 | AAGCCUGCCCGGCUCCUCGGG | 564 |
| hsa-miR-597 | UGUGUCACUCGAUGACCACUGU | 565 |
| hsa-miR-598 | UACGUCAUCGUUGUCAUCGUCA | 566 |
| hsa-miR-599 | GUUGUGUCAGUUUAUCAAAC | 567 |
| hsa-miR-600 | ACUUACAGACAAGAGCCUUGCUC | 568 |
| hsa-miR-601 | UGGUCUAGGAUUGUUGGAGGAG | 569 |
| hsa-miR-602 | GACACGGGCGACAGCUGCGGCCC | 570 |
| hsa-miR-603 | CACACACUGCAAUUACUUUUGC | 571 |
| hsa-miR-604 | AGGCUGCGGAAUUCAGGAC | 572 |
| hsa-miR-605 | UAAAUCCCAUGGUGCCUUCUCCU | 573 |
| hsa-miR-606 | AAACUACUGAAAAUCAAAGAU | 574 |
| hsa-miR-607 | GUUCAAAUCCAGAUCUAUAAC | 575 |
| hsa-miR-608 | AGGGGUGGUGUUGGGACAGCUCCGU | 576 |
| hsa-miR-609 | AGGGUGUUUCUCUCAUCUCU | 577 |
| hsa-miR-610 | UGAGCUAAAUGUGUGCUGGGA | 578 |
| hsa-miR-611 | GCGAGGACCCCUCGGGGUCUGAC | 579 |
| hsa-miR-612 | GCUGGGCAGGGCUUCUGAGCUCCUU | 580 |
| hsa-miR-613 | AGGAAUGUUCCUUCUUUGCC | 581 |
| hsa-miR-614 | GAACGCCUGUUCUUGCCAGGUGG | 582 |
| hsa-miR-615-3p | UCCGAGCCUGGGUCUCCCUCUU | 583 |
| hsa-miR-615-5p | GGGGGUCCCCGGUGCUCGGAUC | 584 |
| hsa-miR-616 | AGUCAUUGGAGGGUUUGAGCAG | 585 |
| hsa-miR-616* | ACUCAAAACCCUUCAGUGACUU | 586 |
| hsa-miR-617 | AGACUUCCCAUUUGAAGGUGGC | 587 |
| hsa-miR-618 | AAACUCUACUUGUCCUUCUGAGU | 588 |
| hsa-miR-619 | GACCUGGACAUGUUUGUGCCCAGU | 589 |
| hsa-miR-620 | AUGGAGAUAGAUAUAGAAAU | 590 |
| hsa-miR-621 | GGCUAGCAACAGCGCUUACCU | 591 |
| hsa-miR-622 | ACAGUCUGCUGAGGUUGGAGC | 592 |
| hsa-miR-623 | AUCCCUUGCAGGGGCUGUUGGGU | 593 |
| hsa-miR-624 | CACAAGGUAUUGGUAUUACCU | 594 |
| hsa-miR-624* | UAGUACCAGUACCUUGUGUUCA | 595 |
| hsa-miR-625 | AGGGGGAAAGUUCUAUAGUCC | 596 |
| hsa-miR-625* | GACUAUAGAACUUUCCCCCUCA | 597 |
| hsa-miR-626 | AGCUGUCUGAAAAUGUCUU | 598 |
| hsa-miR-627 | GUGAGUCUCUAAGAAAAGAGGA | 599 |
| hsa-miR-628-3p | UCUAGUAAGAGUGGCAGUCGA | 600 |
| hsa-miR-628-5p | AUGCUGACAUAUUUACUAGAGG | 601 |
| hsa-miR-629 | UGGGUUUACGUUGGGAGAACU | 602 |
| hsa-miR-629* | GUUCUCCCAACGUAAGCCCAGC | 603 |
| hsa-miR-630 | AGUAUUCUGUACCAGGGAAGGU | 604 |
| hsa-miR-631 | AGACCUGGCCCAGACCUCAGC | 605 |
| hsa-miR-632 | GUGUCUGCUUCCUGUGGGA | 606 |
| hsa-miR-633 | CUAAUAGUAUCUACCACAAUAAA | 607 |
| hsa-miR-634 | AACCAGCACCCCAACUUUGGAC | 608 |
| hsa-miR-635 | ACUUGGGCACUGAAACAAUGUCC | 609 |
| hsa-miR-636 | UGUGCUUGCUCGUCCCGCCCGCA | 610 |
| hsa-miR-637 | ACUGGGGGCUUUCGGGCUCUGCGU | 611 |
| hsa-miR-638 | AGGGAUCGCGGGCGGGUGGCGGCCU | 612 |
| hsa-miR-639 | AUCGCUGCGGUUGCGAGCGCUGU | 613 |
| hsa-miR-640 | AUGAUCCAGGAACCUGCCUCU | 614 |
| hsa-miR-641 | AAAGACAUAGGAUAGAGUCACCUC | 615 |
| hsa-miR-642 | GUCCCUCUCCAAAUGUGUCUUG | 616 |
| hsa-miR-643 | ACUUGUAUGCUAGCUCAGGUAG | 617 |
| hsa-miR-644 | AGUGUGGCUUUCUUAGAGC | 618 |
| hsa-miR-645 | UCUAGGCUGGUACUGCUGA | 619 |
| hsa-miR-646 | AAGCAGCUGCCUCUGAGGC | 620 |
| hsa-miR-647 | GUGGCUGCACUCACUUCCUUC | 621 |
| hsa-miR-648 | AAGUGUGCAGGGCACUGGU | 622 |
| hsa-miR-649 | AAACCUGUGUUGUUCAAGAGUC | 623 |
| hsa-miR-650 | AGGAGGCAGCGCUCUCAGGAC | 624 |
| hsa-miR-651 | UUUAGGAUAAGCUUGACUUUUG | 625 |
| hsa-miR-652 | AAUGGCGCCACUAGGGUUGUG | 626 |
| hsa-miR-653 | GUGUUGAAACAAUCUCUACUG | 627 |
| hsa-miR-654-3p | UAUGUCUGCUGACCAUCACCUU | 628 |
| hsa-miR-654-5p | UGGUGGGCCGCAGAACAUGUGC | 629 |
| hsa-miR-655 | AUAAUACAUGGUUAACCUCUUU | 630 |
| hsa-miR-656 | AAUAUUAUACAGUCAACCUCU | 631 |
| hsa-miR-657 | GGCAGGUUCUCACCCUCUCUAGG | 632 |
| hsa-miR-658 | GGCGGAGGGAAGUAGGUCCGUUGGU | 633 |
| hsa-miR-659 | CUUGGUUCAGGGAGGGUCCCCA | 634 |
| hsa-miR-660 | UACCCAUUGCAUAUCGGAGUUG | 635 |
| hsa-miR-661 | UGCCUGGGUCUCUGGCCUGCGCGU | 636 |
| hsa-miR-662 | UCCCACGUUGUGGCCCAGCAG | 637 |
| hsa-miR-663 | AGGCGGGGCGCCGCGGGACCGC | 638 |
| hsa-miR-665 | ACCAGGAGGCUGAGGCCCCU | 639 |
| hsa-miR-668 | UGUCACUCGGCUCGGCCCACUAC | 640 |
| hsa-miR-671-3p | UCCGGUUCUCAGGGCUCCACC | 641 |
| hsa-miR-671-5p | AGGAAGCCCUGGAGGGGCUGGAG | 642 |
| hsa-miR-672 | UGAGGUUGGUGUACUGUGUGUGA | 643 |
| hsa-miR-674 | GCACUGAGAUGGGAGUGGUGUA | 644 |
| hsa-miR-675 | UGGUGCGGAGAGGGCCCACAGUG | 645 |
| hsa-miR-7 | UGGAAGACUAGUGAUUUUGUUGU | 646 |
| hsa-miR-708 | AAGGAGCUUACAAUCUAGCUGGG | 647 |
| hsa-miR-708* | CAACUAGACUGUGAGCUUCUAG | 648 |
| hsa-miR-7-1* | CAACAAAUCACAGUCUGCCAUA | 649 |
| hsa-miR-7-2* | CAACAAAUCCCAGUCUACCUAA | 650 |
| hsa-miR-744 | UGCGGGGCUAGGGCUAACAGCA | 651 |
| hsa-miR-744* | CUGUUGCCACUAACCUCAACCU | 652 |
| hsa-miR-758 | UUUGUGACCUGGUCCACUAACC | 653 |
| hsa-miR-760 | CGGCUCUGGGUCUGUGGGGA | 654 |
| hsa-miR-765 | UGGAGGAGAAGGAAGGUGAUG | 655 |
| hsa-miR-766 | ACUCCAGCCCCACAGCCUCAGC | 656 |
| hsa-miR-767-3p | UCUGCUCAUACCCCAUGGUUUCU | 657 |
| hsa-miR-767-5p | UGCACCAUGGUUGUCUGAGCAUG | 658 |
| hsa-miR-768-3p | UCACAAUGCUGACACUCAAACUGCUGAC | 659 |
| hsa-miR-768-5p | GUUGGAGGAUGAAAGUACGGAGUGAU | 660 |
| hsa-miR-769-3p | CUGGGAUCUCCGGGGUCUUGGUU | 661 |
| hsa-miR-769-5p | UGAGACCUCUGGGUUCUGAGCU | 662 |
| hsa-miR-770-5p | UCCAGUACCACGUGUCAGGGCCA | 663 |
| hsa-miR-801 | GAUUGCUCUGCGUGCGGAAUCGAC | 664 |
| hsa-miR-802 | CAGUAACAAAGAUUCAUCCUUGU | 665 |
| hsa-miR-871 | UAUUCAGAUUAGUGCCAGUCAUG | 666 |
| hsa-miR-872 | AAGGUUACUUGUUAGUUCAGG | 667 |
| hsa-miR-873 | GCAGGAACUUGUGAGUCUCCU | 668 |
| hsa-miR-874 | CUGCCCUGGCCCGAGGGACCGA | 669 |
| hsa-miR-875-3p | CCUGGAAACACUGAGGUUGUG | 670 |
| hsa-miR-875-5p | UAUACCUCAGUUUUAUCAGGUG | 671 |
| hsa-miR-876-3p | UGGUGGUUUACAAAGUAAUUCA | 672 |
| hsa-miR-876-5p | UGGAUUUCUUUGUGAAUCACCA | 673 |
| hsa-miR-877 | GUAGAGGAGAUGGCGCAGGG | 674 |
| hsa-miR-877* | UCCUCUUCUCCCUCCUCCCAGG | 675 |
| hsa-miR-885-3p | AGGCAGCGGGGUGUAGUGGAUA | 676 |
| hsa-miR-885-5p | UCCAUUACACUACCCUGCCUCU | 677 |
| hsa-miR-886-3p | CGCGGGUGCUUACUGACCCUU | 678 |
| hsa-miR-886-5p | CGGGUCGGAGUUAGCUCAAGCGG | 679 |
| hsa-miR-887 | GUGAACGGGCGCCAUCCCGAGG | 680 |
| hsa-miR-888 | UACUCAAAAAGCUGUCAGUCA | 681 |
| hsa-miR-888* | GACUGACACCUCUUUGGGUGAA | 682 |
| hsa-miR-889 | UUAAUAUCGGACAACCAUUGU | 683 |
| hsa-miR-890 | UACUUGGAAAGGCAUCAGUUG | 684 |
| hsa-miR-891a | UGCAACGAACCUGAGCCACUGA | 685 |
| hsa-miR-891b | UGCAACUUACCUGAGUCAUUGA | 686 |
| hsa-miR-892a | CACUGUGUCCUUUCUGCGUAG | 687 |
| hsa-miR-892b | CACUGGCUCCUUUCUGGGUAGA | 688 |
| hsa-miR-9 | UCUUUGGUUAUCUAGCUGUAUGA | 689 |
| hsa-miR-9* | AUAAAGCUAGAUAACCGAAAGU | 690 |
| hsa-miR-920 | GGGGAGCUGUGGAAGCAGUA | 691 |
| hsa-miR-921 | CUAGUGAGGGACAGAACCAGGAUUC | 692 |
| hsa-miR-922 | GCAGCAGAGAAUAGGACUACGUC | 693 |
| hsa-miR-923 | GUCAGCGGAGGAAAAGAAACU | 694 |
| hsa-miR-924 | AGAGUCUUGUGAUGUCUUGC | 695 |
| hsa-miR-92a | UAUUGCACUUGUCCCGGCCUGU | 696 |
| hsa-miR-92a-1* | AGGUUGGGAUCGGUUGCAAUGCU | 697 |
| hsa-miR-92a-2* | GGGUGGGGAUUUGUUGCAUUAC | 698 |
| hsa-miR-92b | UAUUGCACUCGUCCCGGCCUCC | 699 |
| hsa-miR-92b* | AGGGACGGGACGCGGUGCAGUG | 700 |
| hsa-miR-93 | CAAAGUGCUGUUCGUGCAGGUAG | 701 |
| hsa-miR-93* | ACUGCUGAGCUAGCACUUCCCG | 702 |
| hsa-miR-933 | UGUGCGCAGGGAGACCUCUCCC | 703 |
| hsa-miR-934 | UGUCUACUACUGGAGACACUGG | 704 |
| hsa-miR-935 | CCAGUUACCGCUUCCGCUACCGC | 705 |
| hsa-miR-936 | ACAGUAGAGGGAGGAAUCGCAG | 706 |
| hsa-miR-937 | AUCCGCGCUCUGACUCUCUGCC | 707 |
| hsa-miR-938 | UGCCCUUAAAGGUGAACCCAGU | 708 |
| hsa-miR-939 | UGGGGAGCUGAGGCUCUGGGGGUG | 709 |
| hsa-miR-940 | AAGGCAGGGCCCCCGCUCCCC | 710 |
| hsa-miR-941 | CACCCGGCUGUGUGCACAUGUGC | 711 |
| hsa-miR-942 | UCUUCUCUGUUUUGGCCAUGUG | 712 |
| hsa-miR-943 | CUGACUGUUGCCGUCCUCCAG | 713 |
| hsa-miR-944 | AAAUUAUUGUACAUCGGAUGAG | 714 |
| hsa-miR-95 | UUCAACGGGUAUUUAUUGAGCA | 715 |
| hsa-miR-96 | UUUGGCACUAGCACAUUUUUGCU | 716 |
| hsa-miR-96* | AAUCAUGUGCAGUGCCAAUAUG | 717 |
| hsa-miR-98 | UGAGGUAGUAAGUUGUAUUGUU | 718 |
| hsa-miR-99a | AACCCGUAGAUCCGAUCUUGUG | 719 |
| hsa-miR-99a* | CAAGCUCGCUUCUAUGGGUCUG | 720 |
| hsa-miR-99b | CACCCGUAGAACCGACCUUGCG | 721 |
| hsa-miR-99b* | CAAGCUCGUGUCUGUGGGUCCG | 722 |
| hsv-1 miR-LAT | UGGCGGCCCGGCCCGGGGCC | 723 |

## Claims

1. A bivalent molecule comprising a first oligonucleotide linked to a second oligonucleotide
• wherein the length of the first and the second oligonucleotide is between 7 and 12 nucleotides and at least 50% of the nucleotides of the first and the second oligonucleotide is locked nucleic acid (LNA) monomers
• wherein the first and the second oligonucleotide comprise an antisense sequence complementary to a cellular mRNA or microRNA and the antisense sequences act as steric blockers
• wherein the first oligonucleotide and the second oligonucleotide comprise
i. a seed sequence of microRNA or
ii. a sequence capable of base pairing to the complementary sequence of a seed sequence or
iii. a sequence capable of base pairing to a seed sequence and
• wherein the first and second oligonucleotide is linked via a linking moiety with a length of at least 10 angstrom;
• wherein the linking moiety comprise a non-nucleotide polymer selected from the group consisting of: polyalkylen oxide, polyethyleneglycol , polyacrylic acid, polylactide acid (PLA), poly(glycolic acid) (PGA), polypropylene, polystyrene, polyolefin, polyamide, polycyanoacrylate, polyimide, polyethyleneterephtalat (PEY, PETG), polyethylene terephtalate (PETE), polytetramethylene glycol (PTG) and polyurethane; and
• wherein the first and the second oligonucleotide is not an aptamer, siRNA, ribozyme, RNase H activating antisense oligonucleotide, full unmodified RNA oligonucleotide or full unmodified DNA oligonucleotide and is incapable of recruiting the RNAi machinery and incapable of activating RNase H.

2. The molecule of claim 1, wherein first and the second antisense sequence is a Blockmir antisense sequence capable of binding to a microRNA binding site in a target RNA or wherein first and the second antisense sequence is an antimir antisense sequence capable of binding to a microRNA.

3. The molecule of claim 1 or 2, wherein the first and the second oligonucleotide comprise at least 75 % LNA monomers.

4. The molecule of any of the preceding claims, wherein the linking moiety is incorporated as one or more monomers during standard oligonucleotide synthesis and wherein the monomer adapted for incorporation is selected from the group consisting of: Spacer 18 amidite (17-O-DMT-Hexaethyleneoxide-1-O-phosphoramidite), Spacer 9 Amidite (8-DMT-O-Triethyleneoxide-1-O-phosphoramidite), C6 Spacer Amidite (6-DMT-O-Hexanediol-1-O-Phosphoramidite) and C3 Spacer Amidite (DMT-1,3 propanediol-phosphoramidite).

## Patentansprüche

1. Ein zweiwertiges Molekül, umfassend ein erstes Oligonukleotid, das mit einem zweites Oligonukleotid verbunden ist,
• wobei die Länge des ersten und zweiten Oligonukleotids zwischen 7 und 12 Nukleotiden beträgt und mindestens 50% der Nukleotide des ersten und zweite Oligonukleotids fixierte Nukleinsäure (*locked nucleic acids,* LNA)-Monomere sind
• wobei das erste und das zweite Oligonukleotid eine Sequenz mit (-)-Polarität (*antisense sequence)* umfassen, die komplementär zu einer zellulären mRNA oder microRNA ist und die Sequenzen mit (-)-Polarität als sterische Inhibitoren wirken
• wobei das erste und das zweite Nukleotid umfassen
i. eine Kernsequenz (*seed sequence*) einer microRNA oder
ii. eine Sequenz, die mit der zu einer Kernsequenz komplementären Sequenz Basenpaarung eingehen kann oder
iii. eine Sequenz, die mit einer Kernsequenz Basenpaarung eingehen kann
• wobei das erste und das zweite Oligonukleotid über eine Verknüpfungseinheit mit einer Länge von mindestens 10 Angström verknüpft ist,
• wobei die Verknüpfungseinheit ein Nicht-Nukleotid-Polymer ausgewählt aus der Gruppe bestehend aus Polyalkylenoxid, Polyethylenglcyol, Polyacrylsäure, Polymilchsäure (PLA), poly(Glykolsärure) (PGA), Polypropylen, Polystyren, Polyolefin, Polyamid, Polycyanoacrylat, Polyimid, Polyethylenetherterephtalat (PEY, PETG), Polyethylenterephtalat (PETE), Polytetramethylen-Glycol (PTG) und Polyurethan umfasst; und
• wobei das erste und das zweite Oligonucleotid kein Aptamer, siRNA, Ribozym, RNase H-aktivierendes Antisense-Oligonukleotid, vollständiges unmodifiziertes RNA-Oligonukleotid oder vollständiges unmodifiziertes DNA-Oligonukleotid sind und unfähig sind, die RNAi-Maschinerie zu rekrutieren und unfähig sind, RNase H zu aktivieren

2. Molekül nach Anspruch 1, wobei die erste und die zweite Sequenz mit (-)-Polarität eine Blockmir-Antisense-Sequenz ist, die in der Lage ist, an eine microRNA-Bindungsstelle in einer Ziel-RNA zu binden oder wobei die erste und die zweite Antisense-Sequenz eine Antimir-Antisense-Sequenz sind, die in der Lage ist, an eine microRNA zu binden.

3. Molekül nach Anspruch 1 oder 2, wobei das erste und das zweite Oligonukleotid mind. 75% LNA-Monomere umfassen.

4. Molekül nach einem der vorhergehenden Ansprüche, wobei die Verknüpfungseinheit als ein oder mehrere Monomere während der Standard-Oligonukleotidsynthese eingebaut ist und wobei das Monomer, das für den Einbau geeignet ist, ausgewählt ist aus der Gruppe bestehend aus: Spacer 18 Amidit (17-O-DMT-Hexaethylenoxid-1-O-phosphoramidit), Spacer 9 Amidit (8-DMT-O-Triethylenoxid-1-O-phosphoramidit), C6 Spacer Amidit (6-DMT-O-Hexandiol-1-O-phosphoramidit) und C3 Spacer Amidit (DMT-1,3-propandiol-phosphoramidit).

## Revendications

1. Une molécule bivalent comprenant un premier oligonucléotide relié à un deuxième oligonucléotide,
• dans lequel la longueur du premier et du deuxième oligonucléotide est 7 à 12 nucléotides, et au moins 50% des nucléotides du premier et deuxième oligonucléotide sont des monomères des acides nucléiques bloqués (*locked nucleic acid,* LNA),
• dans lequel le premier et le deuxième oligonucléotide comprennent une séquence antisens complémentaire à un ARNm cellulaire ou à un microARN et les séquences antisens agissent comme inhibiteurs stériques
• dans lequel le premier et le second nucléotide comprennent
i. une séquence de base (seed region) d'un microARN
ii. une séquence capable d'appariement des bases avec la séquence complémentaire d'une séquence de base ou
iii. une séquence capable d'appariement de bases avec une séquence de base
• dans lequel le premier et le deuxième oligonucléotide sont liés par un groupement de liaison ayant une longueur d'au moins 10 Angstroms,
• dans lequel le groupement de liaison est un polymère non-nucléotidique choisie dans le groupe consistant de l'oxyde de polyalkylène, de polyéthylèneglcyol, de l'acide polyacrylique, de l'acide polylactique (PLA), du poly (acide de glycole) (PGA), du polypropylène, du polystyrène, de la polyoléfine, du polyamide, du polycyanoacrylate, du polyimide, du téréphtalate d'éther de polyéthylène (PEY, PETG), du téréphtalate de polyéthylène (PETE), du polytétraméthylène glycol (PTG), et du polyuréthanne; et
• dans lequel le premier et le deuxième oligonucléotide ne sont pas un aptamère, un ARNsi, un ribozyme, un oligonucléotide antisens activant la RNase H, un oligonucléotide d'ARN complet non-modifié ou un oligonucleotide complet d'ADN non-modifiée et sont incapable de recruter la machinerie d'ARNi et sont incapable d'activer la RNase H.

2. Molécule selon la revendication 1, dans lequel la première et la deuxième séquence antisens est une séquence antissens Blockmir capable de se lier à un site de liaison ARNmi dans un ARN cible, ou dans lequel la première et la séquence antisens est une séquence antisens antimir capable de se lier à un ARNmi.

3. Molécule selon la revendication 1 ou 2, dans lequel le premier et le deuxième oligonucléotide comprennent au moins 75% de monomères de LNA.

4. Molécule selon l'une des revendications précédentes, dans lequel le groupement de liaison est constituée en un ou plusieurs monomères au cours de la synthèse d'oligonucléotides standard, et dans lequel le monomère qui est approprié pour l'incorporation est choisi dans le groupe consistant de: Spacer 18 amidite (17- O-DMT-hexaéthylèneoxyde-1-O-phosphoramidite), Spacer 9 amidite (8-DMT-O-triéthylèneoxyde-1-O-phosphoramidite), C6 Spacer amidite (6-DMT-O-hexanediol-1-O-phosphoramidite) et C3 Spacer amidite (DMT-1,3-propanediol-phosphoramidite).
